# EUROPEAN PATENT APPLICATION

(11) **EP 2 586 479 A1**
(43) Date of publication of application: **01.05.2013**
(21) Application number: 11187237.0
(22) Date of filing: 31.10.2011
(51) Int. Cl.: A61M 5/32

(54) **Safety needle assembly**

(71) Applicant: Sanofi-Aventis Deutschland GmbH, 65929 Frankfurt am Main (DE)
(72) Inventor: The designation of the inventor has not yet been filed

(57) **Abstract**

Described is a safety needle assembly (1, 101 to 901) comprising a needle hub (2, 102 to 902) mounting a needle (3, 103 to 903), a needle shield (4, 104 to 904) translatably arranged with respect to the needle hub (2, 102 to 902) and comprising a distal base member (4.2, 104.2 to 904.2) with a needle aperture (4.3, 104.3 to 904.3) through which a needle (3, 103 to 903) may pass, and a closure element (7, 107 to 907) moveably arranged with respect to the needle shield (4, 104 to 904). When the needle shield (4, 104 to 904) is moved from a retracted position (PR, PR101 to PR901) to an advanced position (PA, PA101 to PA901) the closure element (7, 107 to 907) is moved in such a way that it closes the needle aperture (4.3, 104.3 to 904.3).

## Description

### Technical Field

The present invention relates to a safety needle assembly that may be mounted to a housing of a drug delivery device. The safety needle assembly comprises a needle and a needle shield for covering the needle to reduce the risk of accidental needle stick injuries. Moreover, the present invention relates to a drug delivery device with a safety needle assembly.

### Background of the Invention

Drug delivery devices that contain a selected dosage of a medicament or drug are well known devices for administering the medicament to a patient. Safety devices for covering a needle of the drug delivery device before and after use are also well known. Typically, a needle shield of the safety device is either manually moved or translated by the action of a relaxing spring to surround the needle.

A different type of safety devices known in the state of the art solve the object of providing needle safety by arranging the needle movable relative to a body, wherein a syringe with the needle attached thereto is retracted into the body after drug delivery.

In particular, drug delivery devices known in the state of the art as pen injectors or auto-injectors comprise a cartridge containing the medicament or the drug. A safety needle comprises a needle hub with the needle attached thereto and a needle shield. The safety needle assembly is typically detachably mounted to a housing of the drug delivery device. The needle is covered by a needle shield of the safety device at least after the drug has been delivered so as to avoid needle stick injuries with used needles.

It is an object of the present invention to provide an improved safety needle assembly suitable to be mounted to a housing of a drug delivery device that minimizes the risk of accidental needle stick injuries.

It is a further object of the present invention to provide an improved drug delivery device that is safe to handle.

The object is achieved by a safety needle assembly according to claim 1 and by a drug delivery device according to claim 24.

Preferred embodiments of the invention are given in the dependent claims.

In an exemplary embodiment, a safety needle assembly according to the present invention comprises a needle hub mounting a needle, a needle shield translatably arranged with respect to the needle hub and comprising a distal base member with a needle aperture through which the needle may pass, and a closure element moveably arranged with respect to the needle shield. When the needle shield is moved from a retracted position to an advanced position, the closure element is moved in such a way that it closes the needle aperture.

In an exemplary embodiment, the closure element is arranged inside the safety needle assembly between at least the needle shield and the needle hub. The closure element is rotatably arranged with respect to the needle shield between an unclosed position) and a closed position.

In an exemplary embodiment, the closure element is designed as a transverse flap which is pivoted about a longitudinal axis from the unclosed position to the closed position. The transverse flap comprises an integrated spring arm which biases the transverse flap relative to a release sleeve and the needle shield) in a compressed state so as to releasably retain the transverse flap in the unclosed position. When the release sleeve is released upon translation of the needle hub relative to the needle shield, the spring arm relaxes to pivot the transverse flap to the closed position in which the needle aperture is closed.

In an exemplary embodiment, the transverse flap is pivotably mounted on a needle shield. Upon translation of the needle hub relative to the needle shield, a rotation sleeve locks in corresponding snaps of the needle hub so that the rotation sleeve is axially moved with the needle hub and the flap is pivoted by being guided in an inclined slot of the rotation sleeve to close the needle aperture.

In an exemplary embodiment, the closure element is designed as at least one flap which is pivoted about a transversal axis from the unclosed position to the closed position. The flap is pivotably mounted on a slider whereby upon translation of the needle hub relative to the needle shield the slider locks in corresponding snaps of the needle hub so that the slider is axially moved with the needle hub. The flap is pivoted inwards so that the distal end of the flap closes the needle aperture.

In an exemplary embodiment, the closure element is designed as at least one blocking arm which is pivoted about a transversal axis from the unclosed position to the closed position and vice versa. Upon translation of the needle hub relative to the needle shield in the distal direction, an interference arm arranged on the needle hub flexes the blocking arm out of the needle aperture to open it, and upon translation of the needle hub relative to the needle shield in the proximal direction, the blocking arm flexes back over the needle aperture to irreversibly close it. The interference arm is guided and finally locked in a guiding slot of the blocking arm.

In an exemplary embodiment, the closure element is designed as a wing flap with opposite mounted wings which are pivoted about respective transversal axes from the unclosed position to the closed position. Each wing of the wing flap is guided in an essentially longitudinal direction on a locking arm of a U-shaped locking clamp whereby upon translation of the needle hub relative to the needle shield proximal ends of the locking arms lock in corresponding snaps of the needle hub so that the locking clamp is axially moved with the needle hub. The wings are axially guided on an inner surface of the locking arms and on a distal inclined section of the inner surface of the locking arms the opposite wings are pivoted so that the ends of the opposite wings engage each other to close the needle aperture.

In an exemplary embodiment, the closure element is designed as a ball bearing which is guided from the unclosed position to the closed position. The ball bearing is retained by a flip mechanism relative to the needle shield in the unclosed position whereby when the flip mechanism is released upon translation of the needle hub relative to the needle shield a release flap of the flip mechanism is pivoted and releases a ball of the ball bearing so that the ball is guided to the closed position in which the needle aperture is closed. The ball bearing is rotatably retained above the needle aperture, whereby the ball of the ball bearing comprises a through hole for the needle and is rotated and guided upon translation of the needle hub relative to the needle shield by actuator fingers arranged on an actuator sleeve which is coupled and axially moved with the needle hub to rotate the ball such that the through hole is misaligned with respect to the needle aperture.

In an exemplary embodiment, the closure element is designed as an inner sleeve which is arranged between the needle shield and the needle hub and which is rotatable about a longitudinal axis with respect to the needle shield from an angular position in which the needle aperture is opened to another angular position in which the needle aperture is closed. The inner sleeve and the needle shield are concentrically arranged about a longitudinal axis and each of them comprise needle apertures, wherein the needle apertures and the needle are eccentrically arranged with respect to the longitudinal axis.

In an exemplary embodiment, the closure element is drawably arranged with respect to the needle shield between an unclosed position and a closed position. The closure element is designed as a flexible strip which is drawably arranged on an inner surface of the needle shield. When the needle hub is relatively moved to the needle shield the flexible strip is drawn from the unclosed position to the closed position in which the needle aperture is closed by an essentially transversally guided section of the flexible strip. At least a proximal end of the flexible strip and at least a distal end of the needle hub comprise corresponding snap-fit elements and the opposite end of the flexible strip and a guiding track in the needle shield comprise corresponding fixed stop elements to irreversibly lock the flexible strip with respect to the needle shield (104) in the closed position.

In an exemplary embodiment, the closure element is designed as a spring cover which is arranged between the needle shield and the needle hub and which is axially movable with respect to the needle shield or the needle hub from the unclosed position to the closed position in which a clearance in the needle shield allows the spring cover to relax and close the needle aperture. An inner surface of the needle shield comprises an inclined section. Upon translation of the needle hub relative to the needle shield the spring cover is stressed by being guided on the inclined section of the needle shield.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: an isometric and view of a safety needle assembly according to a first embodiment of the invention;
- Figure 2: a first sectional view of the safety needle assembly according to the first embodiment before use;
- Figure 3: a second sectional view of the safety needle assembly according to the first embodiment before use;
- Figure 4: a sectional view of the safety needle assembly according to the first embodiment during use;
- Figure 5: a first sectional view of the safety needle assembly according to the first embodiment after use in a needle safe state;
- Figure 6: a second sectional view of the safety needle assembly according to the first embodiment after use in a needle safe state;
- Figure 7: an isometric and sectional view of a safety needle assembly according to a second embodiment of the invention before use;
- Figure 8: an isometric and sectional view of the safety needle assembly according to the second embodiment of the invention during use;
- Figure 9: an isometric and sectional view of the safety needle assembly according to the second embodiment of the invention after use in a needle safe state;
- Figure 10: an isometric and sectional view of a safety needle assembly according to a third embodiment of the invention before use;
- Figure 11: an isometric and sectional view of the safety needle assembly according to the third embodiment of the invention during use;
- Figure 12: an isometric and sectional view of the safety needle assembly according to the third embodiment of the invention after use in a needle safe state;
- Figure 13: an isometric and sectional view of a safety needle assembly according to a fourth embodiment of the invention before use;
- Figure 14: an isometric and sectional view of the safety needle assembly according to the fourth embodiment of the invention during use;
- Figure 15: an isometric and sectional view of the safety needle assembly according to the fourth embodiment of the invention after use in a needle safe state;
- Figure 16: an isometric and sectional view of a safety needle assembly according to a fifth embodiment of the invention before use;
- Figure 17: an isometric and sectional view of the safety needle assembly according to the fifth embodiment of the invention during use;
- Figure 18: an isometric and sectional view of the safety needle assembly according to the fifth embodiment of the invention after use in a needle safe state;
- Figure 19: an isometric and sectional view of a safety needle assembly according to a sixth embodiment of the invention before use;
- Figure 20: an isometric and sectional view of the safety needle assembly according to the sixth embodiment of the invention during use;
- Figure 21: an isometric and sectional view of the safety needle assembly according to the sixth embodiment of the invention after use in a needle safe state;
- Figure 22: an isometric and sectional view of a safety needle assembly according to a seventh embodiment of the invention before use;
- Figure 23: an isometric and sectional view of the safety needle assembly according to the seventh embodiment of the invention during use;
- Figure 24: an isometric and sectional view of the safety needle assembly according to the seventh embodiment of the invention after use in a needle safe state;
- Figure 25: an isometric and sectional view of a safety needle assembly according to an eighth embodiment of the invention before use;
- Figure 26: a sectional view of a safety needle assembly according to an eighth embodiment of the invention before use;
- Figure 27: an isometric and sectional view of the safety needle assembly according to the eighth embodiment of the invention during use;
- Figure 28: an isometric and sectional view of the safety needle assembly according to the eighth embodiment of the invention after use in a needle safe state;
- Figure 29: an isometric and sectional view of a safety needle assembly according to a ninth embodiment of the invention before use;
- Figure 30: an isometric and sectional view of the safety needle assembly according to the ninth embodiment of the invention during use;
- Figure 31: an isometric and sectional view of the safety needle assembly according to the ninth embodiment of the invention after use in a needle safe state;
- Figure 32: an isometric and sectional view of a safety needle assembly according to a tenth embodiment of the invention before use;
- Figure 33: an isometric and sectional view of the safety needle assembly according to the tenth embodiment of the invention during use;
- Figure 34: an isometric and sectional view of the safety needle assembly according to the tenth embodiment of the invention after use in a needle safe state;

Corresponding parts are marked with the same reference symbols in all figures.

### Detailed Description

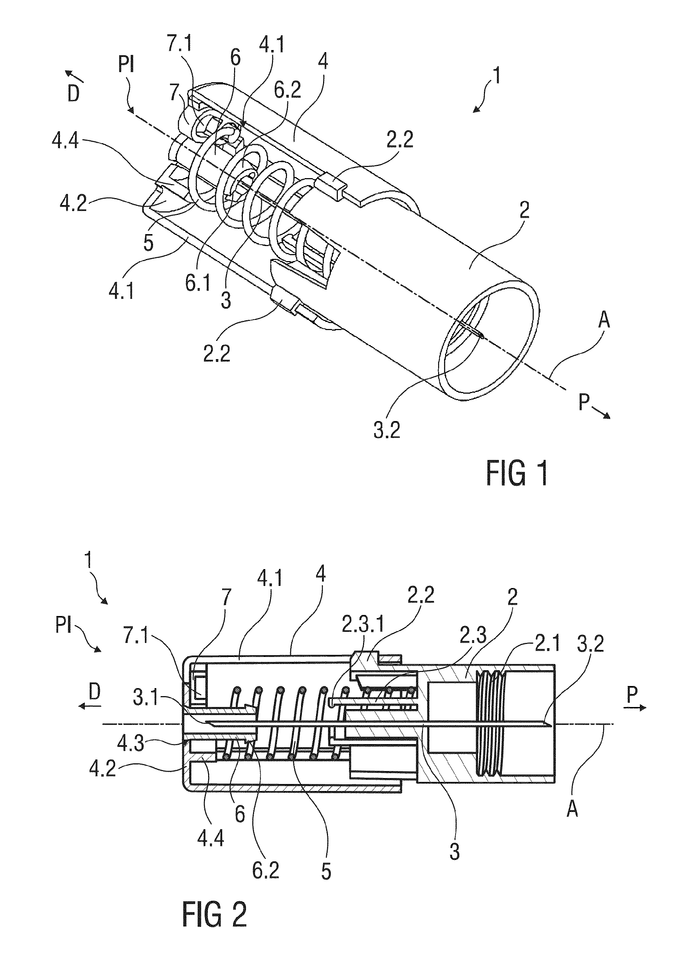

Figures 1 and 2 show sectional and isometric views of a safety needle assembly 1 according to a first embodiment of the invention before use. The safety needle assembly 1 comprises a needle hub 2 mounting a needle 3 and a substantially cylindrical needle shield 4 translatably arranged on the needle hub 2. For illustrative purposes, parts of the needle shield 4 surrounding the inner mechanism of the safety needle assembly 1 are not shown in figure 1.

The safety needle assembly 1 is arranged in a state as it would be presented to an end-user. The needle shield 4 is positioned in a first axial position, e.g. in an initial advanced position PI, wherein a pointed distal tip 3.1 of the needle 3 is surrounded by the needle shield 4.

The needle shield 4 is axially translatable relative to the needle hub 2 so as to cover and expose the distal tip 3.1 of the needle 3 when a drug is delivered to a patient. The safety needle assembly 1 is adapted to be mounted to a housing of a drug delivery device (not illustrated) containing the drug or medicament. According to possible embodiments of the invention, the drug delivery device may be arranged as a syringe, a dental syringe, an auto-injector, a pen injector or a similar device suitable for delivering the drug or the medicament to the patient.

In the exemplary embodiment shown, a thread 2.1 is arranged on the inside of a cylindrical surface of the needle hub 2 and adapted to engage a corresponding thread arranged on the housing of the drug delivery device. Alternatively, the safety needle assembly 1 may be mounted to the drug delivery device by a bayonet type coupling, a snap fit coupling or another suitable coupling that allows for removal of the safety needle assembly 1 after use.

Alternatively, the needle hub 2 or the needle 3 of the safety needle assembly 1 may be arranged with the housing of the drug delivery device as one piece.

The needle 3 is arranged as a double pointed needle having the pointed distal tip 3.1 and a pointed proximal tip 3.2 protruding in a proximal direction P. The proximal tip 3.2 of the needle 3 is arranged to be inserted into a cartridge or container containing the drug or medicament when the safety needle assembly 1 is mounted to the housing of the drug delivery device. The exemplary embodiment shown is thus particularly suited to be used in combination with a reusable drug delivery device that is adapted to receive the container or cartridge such as a pen injector.

Three radial protrusions 2.2 are arranged around an outer circumference of the needle hub 2 and at equal distances to each other. Each radial protrusion 2.2 extends radial outwards from the needle hub 2 and protrudes through a corresponding longitudinal slot 4.1 formed into the needle shield 4. The longitudinal slot 4.1 extends parallel to an axis A of the substantially cylindrical needle hub 2 and needle shield 4. The radial protrusion 2.2 travels along the longitudinal slot 4.1 when the needle shield 4 is translated relative to the needle hub 2 so as to prevent a rotation of the needle shield 4 relative to the needle hub 2.

A spring element 5 arranged as a compression spring biases the needle shield 4 with respect to the needle hub 2 in a distal direction D.

The needle shield 4 has a first base member 4.2 substantially covering the open distal end of the needle shield 4. The base member 4.2 has a needle aperture 4.3 concentrically arranged about the longitudinal axis A.

A release sleeve 6 frictionally engages the needle aperture 4.3 and is releasably retained in the needle aperture 4.3. The release sleeve 6 has a substantially cylindrical shape with a lateral wall having a longitudinal through cut 6.1 that allows for a slight deformation of the release sleeve 6 so that the release sleeve 6 may disengage the needle aperture 4.3 when pulled in the proximal direction P.

The release sleeve 6 further comprises a proximal flange 6.2 that projects radially outwards therefrom. At least one longitudinal first arm 2.3 protrudes from the needle hub 2 in the distal direction D that is adapted to latch to the proximal flange 6.2 so that the retention of the release sleeve 6 in the needle aperture 4.3 may be released by translating the needle hub 2 with respect to the needle shield 4 in the proximal direction P during use of the safety needle assembly 1. Especially the distal end of the first arm 2.3 comprises a snap 2.3.1 which corresponds to the proximal flange 6.2 of the release sleeve 6.

The needle 3 is arranged to protrude through the needle aperture 4.3 when the needle shield 4 is translated from the initial advanced position PI in the proximal direction P.

The safety needle assembly 1 comprises a closure element 7 which is moveably arranged with respect to the needle shield 4.

The closure element 7 is arranged to cover the needle aperture 4.3 after use of the safety needle assembly 1 so as to block a subsequent exposure of the needle 3. The safety needle assembly 1 thus minimizes the risk that the used needle 3 is exposed, so that a transmission of blood-borne diseases, like for example HIV, AIDS, Hepatitis B or Hepatitis C may be avoided.

According to a first embodiment the closure element 7 comprises a transverse flap 7.1 that is pivotably arranged on the base member 4.2 of the needle shield 4.

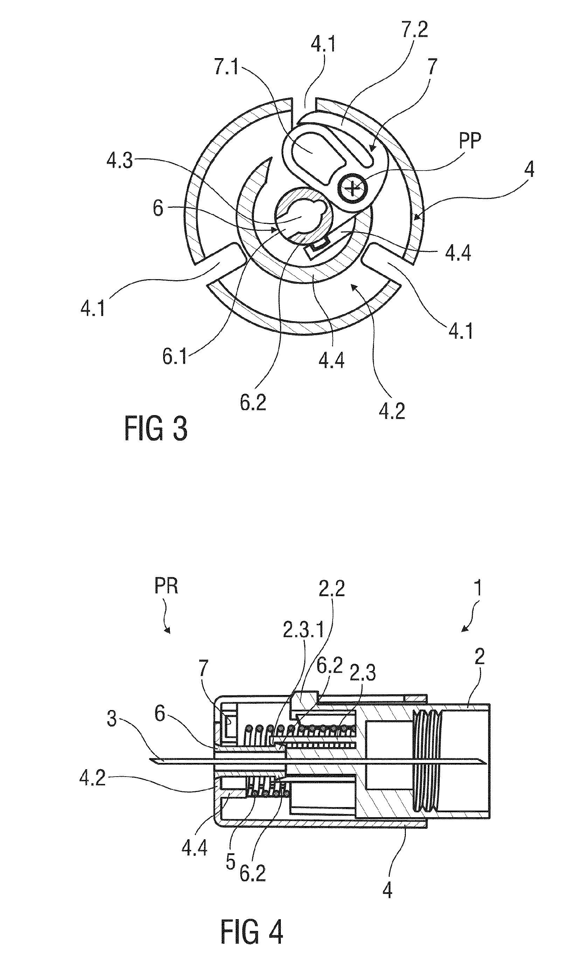

Figure 3 shows a second sectional view of the safety needle assembly 1 before use, wherein the sectional plane shown extends perpendicularly to the one shown in the first sectional view of figure 2.

A biasing element shaped as a spring arm 7.2 is arranged with the closure element 7 as one piece. The closure element 7 integrally formed with the spring arm 7.2 is made from a plastic material of suitable resiliency so as to provide a rotational bias for the transverse flap 7.1 about a pivot point PP.

Before use, the release sleeve 6 abuts laterally against the transverse flap 7.1 and an inner surface of the needle shield 4 so as to retain the spring arm 7.2 in a compressed and energized state in an unclosed position PU in which the needle aperture 4.3 is not covered by the transverse flap 7.1. The spring arm 7.2 is arranged to unfold upon removal of the release sleeve 6 from the needle aperture 4.3 so as to pivot the transverse flap 7.1 about the pivot point PP.

The base member 4.2 has a bearing surface 4.4 against which the transverse flap 7.1 abuts so that the transverse flap 7.1 comes to a rest in a position in which the needle aperture 4.3 of the base member 4.2 is covered.

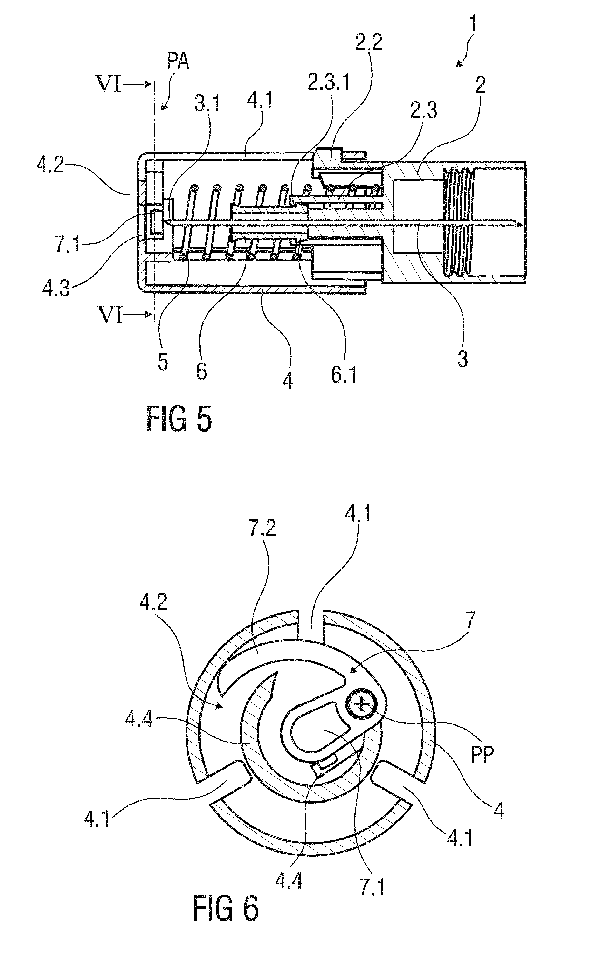

Figure 4 shows a sectional view of the safety needle assembly 1 according to the first embodiment of the invention during use. The needle 3 protrudes from the needle shield 4 in the distal direction D that is retained in another axial position, e.g. in a retracted PR. The spring element 5 is fully compressed and energized so as to bias the needle shield 4 and the needle hub 2 away from each other. The snap 2.3.1 of the longitudinal arm 2.3 engages the proximal flange 6.2 of the release sleeve 6 so that the release sleeve 6 may brought out of engagement with the needle aperture 4.3 by translating the needle shield 4 with respect to the needle hub 2 from the retracted position PR towards a third axial position, e.g. an safe advanced position PA illustrated in more detail in figure 5.

Figure 5 shows a first sectional view of the safety needle assembly 1 in a needle safe state after the drug has been delivered. The distal tip 3.1 of the needle 3 is covered and shielded by the needle shield 4 positioned in the advanced position PA.

The release sleeve 6 has been removed from the needle aperture 4.3. The transverse flap 7.1 of the closure element 7 covers the needle aperture 4.3 so as to block a subsequent exposure of the needle 3.

Figure 6 shows a second sectional view of the safety needle assembly 1 in the needle safe state, wherein the sectional plane shown extends perpendicularly to the one of the first sectional view illustrated in figure 5. The spring arm 7.2 is unfolded and bears against the inner surface of the needle shield 4 so as to push the transverse flap 7.1 from the unclosed position PU to the closed position PC against the bearing surface 4.4 and retain the transverse flap 7.1 over the needle aperture 4.3 so that the needle aperture 4.3 is covered and closed.

A drug delivery process involving the safety needle assembly 1 may be carried out as follows: in a first step, the safety needle assembly 1 is attached to a distal end of a housing of a drug delivery device. Initially, the needle shield 4 is retained in the initial advanced position PI as illustrated in figures 1, 2 and 3.

The safety needle assembly 1 mounted to the drug delivery device is then arranged on the skin of the patient in a manner that the longitudinal axis A extends essentially perpendicularly to the skin of the patient. The base member 4.2 rests on the skin of the patient so that the needle shield 4 may be axially translated from the initial advanced position PI in the proximal direction P by pushing the drug delivery device towards the skin surface until the proximal flange 6.2 of the release sleeve 6 abuts on a distal end 2.4 of the needle hub 2, whereby the torsion spring 5 is partially tensioned and the at least one snap 2.3.1 of the longitudinal arm 2.3 protrudes the distal end 2.4 of the needle hub 2 and locks in the corresponding proximal flange 6.2 of the release sleeve 6.

The needle shield 4 is now arranged in the retracted position PR as illustrated in figure 4. The needle 3 protrudes from a distal end of the safety needle assembly 1 by a length that allows for a disposal of the drug in an appropriate depth. The torsion spring 5 is compressed and energized so that the needle shield 4 automatically advances towards the advanced position PA illustrated in figure 5 when the drug delivery device with the safety needle assembly 1 mounted thereto is removed from the skin surface of the patient. The needle hub 2 together with the release sleeve 6 now translates relative to the needle shield 4 until the radial protrusions 2.2 of the needle hub 2 engages the proximal end of the slots 4.1 of the needle shield 4.

Upon this translation of the needle shield 4 from the retracted position PR to the advanced position PA in the distal direction D, the spring arm 7.2 of the closure element 7 relaxes and unfolds so as to pivot the transverse flap 7.1 about the pivot point PP and to push the transverse flap 7.1 from the unclosed position PU to the closed position PC against the bearing surface 4.4 and retain the transverse flap 7.1 over the needle aperture 4.3 so that the needle aperture 4.3 is covered and closed.

The safety needle assembly 1 is in a needle safe state when the needle shield 4 reaches the advanced position PA as shown in figure 5. The needle shield 4 surrounds the needle 3 so as to reduce the risk of an accidental needle stick injury caused by the used and thus contaminated needle 3. The safety needle assembly 1 may thus also help to reduce transmission of blood-borne diseases, like for example HIV, AIDS, Hepatitis B or Hepatitis C.

The transverse flap 7.1 is irreversibly positioned over the needle aperture 4.3 and the release sleeve 6 is fully withdrawn. Thus, a subsequent proximal translation of the needle shield 4 with respect to the needle hub 2 is blocked. The safety needle assembly 1 is thus irreversibly locked in the advanced position PA and may be safely detached from the drug delivery device 1 and disposed after use.

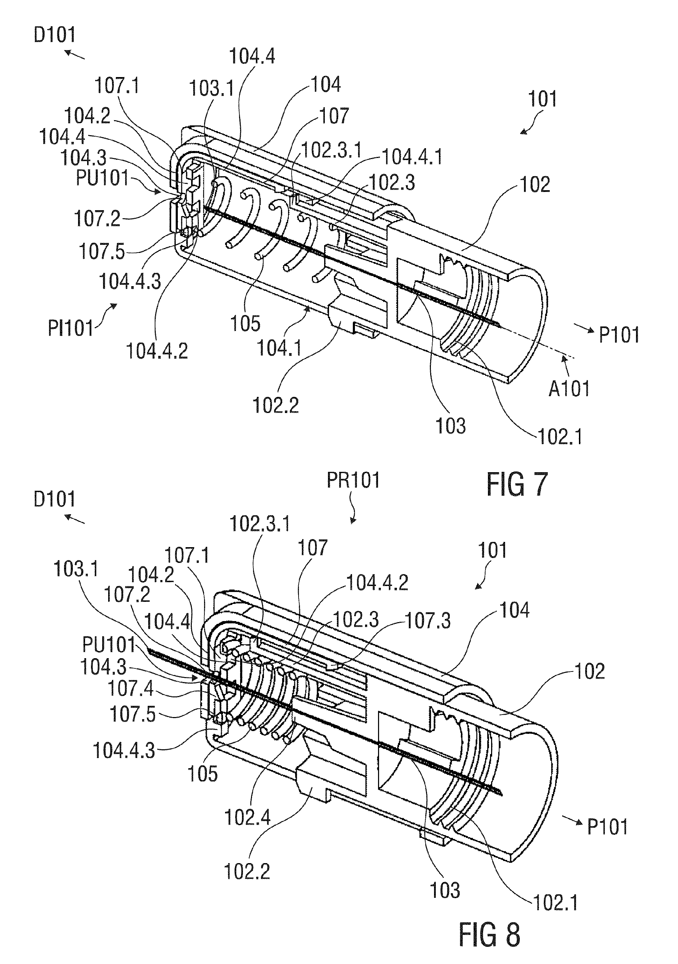

Figures 7 to 9 show sectional and isometric views of a safety needle assembly 101 according to a second embodiment of the invention before, during and after use. The safety needle assembly 101 comprises a needle hub 102 mounting a needle 103 and a substantially cylindrical needle shield 104 translatably arranged on the needle hub 102.

In figure 7, the safety needle assembly 101 is arranged in a state as it would be presented to an end-user. The needle shield 104 is positioned in an initial advanced position PI101, wherein a pointed distal tip 103.1 of the needle 103 is surrounded by the needle shield 104.

The needle shield 104 is axially translatable relative to the needle hub 102 so as to cover and expose the distal tip 103.1 of the needle 103 when a drug is delivered to a patient (shown in figure 8).

Furthermore, the needle hub 102 is designed as in the previous embodiment shown in figures 1 to 6 and also comprises three radial protrusions 102.2 arranged around an outer circumference of the needle hub 102 and at equal distances to each other. The needle shield 104 comprises corresponding longitudinal slots 104.1 formed into the wall of the needle shield 104. The radial protrusion 102.2 travels along the longitudinal slot 104.1 when the needle shield 104 is translated relative to the needle hub 102 so as to prevent a rotation of the needle shield 104 relative to the needle hub 102.

A spring element 105 arranged as a compression spring biases the needle shield 104 with respect to the needle hub 102 in a distal direction D101.

The needle shield 104 has a base member 104.2 substantially covering the open distal end of the needle shield 104. The base member 104.2 has a needle aperture 104.3 concentrically arranged about the longitudinal axis A101.

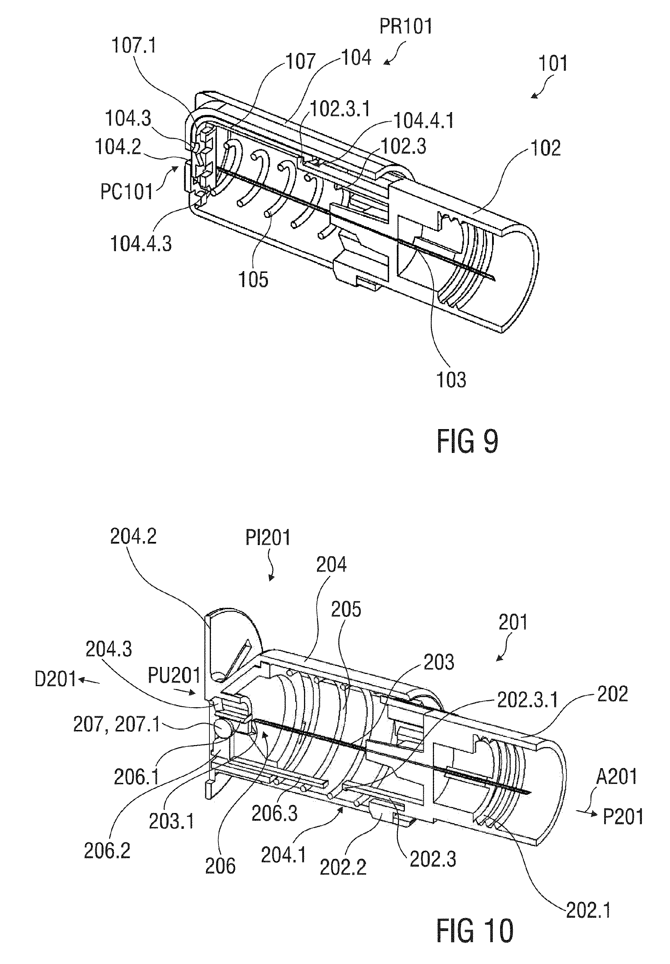

Furthermore, the safety needle assembly 101 comprises an alternative closure element 107. The closure element 107 according to the second embodiment is drawably arranged with respect to the needle shield 104 between an unclosed position PU101 in which the needle aperture 104.3 is uncovered and opened (shown in figures 7 and 8) and a closed position PC101 in which the needle aperture 104.3 is covered and closed by the closure element 107 (shown in figure 9).

The closure element 107 is designed as a flexible strip 107.1. The flexible strip 107.1 is made from a plastic material of suitable resiliency so as to provide a flexing of a section of the flexible strip 107.1 and which is also resilient to needle penetration. Especially the flexible strip 107.1 is made from thermoplastic polymer, e.g. polypropylene.

For guiding the flexible strip 107.1 the needle shield 104 comprises on an inner surface an integrated guiding track 104.4. The flexible strip 107.1 comprises a through hole 107.2 for the needle 103 which is aligned with the needle aperture 104.3 at least in the retracted position PR101 of the needle shield 104 (shown in figure 8). Furthermore, the flexible strip 107.1 comprises a snap-fit element 107.3 at a proximal end which protrudes through a first recess 104.4.1 at the proximal end of the guiding track 104.4. The snap-fit element 107.3 corresponds with a snap 102.3.1 on a distal end 102.4 of the needle hub 102. The snap-fit element 107.3 of the flexible strip 107.1 has a hook-like design and is adapted to latch the snap 102.3.1 of the needle hub 102 so that the flexible strip 107.1 may be drawn in the proximal direction P101 when the needle hub 102 relatively translates to the needle shield 104 during use of the safety needle assembly 101.

Further, the first recess 104.4.1 is adapted such that the snap 102.3.1 of the needle hub 102 is irreversibly locked in the first recess 104.4.1 with respect to the needle shield 104 in the closed position PC101 of the flexible strip 107.1 (shown in figure 9) in which the needle aperture 104.3 is covered by an essentially transversally guided section of the flexible strip 107.1 so that the needle 103 is unable to pass through the needle shield 104 and the safety needle assembly 101 can not be used a second time. Hence the snap-fit connection of the needle shield 104 and the needle hub 102 at the proximal end of the integrated guiding track 104.4 introduces a no-return feature so that the safety needle assembly 101 may only be used once and may be safely detached from a drug delivery device and disposed after use. Needle stick injuries with contaminated needles 103 may be avoided by the locking flexible strip 107.1.

Because the flexible strip 107.1 runs in the integrated guiding track 104.4 with a bent section of approximately 90° the material of the flexible strip 107.1 is made from a resilient plastics material allowing a limited elastic deformations and which is adapted to smoothly slide in the guiding track 104.4. Preferably the flexible strip 107.1 is made from polypropylene.

The opposite distal end of the flexible strip 107.1 and the distal end of the guiding track 104.4 comprise corresponding latching elements which are respectively designed as at least one needle safety element 107.4 designed as wings with a greater material thickness to prevent needle penetration. The wings 107.4 are arranged on an outer surface of the flexible strip 107.1 and the further recesses 104.4.2 in the wall of the integrated guiding track 104.4. Furthermore, the integrated guiding track 104.4 comprises at least one fixed stop 104.4.3 with an inclined section at its distal end. The inclined section serves for energizing the flexible wings 107.5 at the distal end of the flexible strip 107.1 as the flexible strip 107.1 is drawn into the proximal direction P101 .The flexible wings 107.5 then expand in one of the recesses 104.4.2 so that the flexible strip 107.1 is locked in the integrated guiding track 104.4.

A drug delivery process involving the safety needle assembly 101 may be carried out as follows: in a first step, the safety needle assembly 101 is attached to a distal end of a housing of a drug delivery device. Initially, the needle shield 104 is retained in the initial advanced position PI101 as illustrated in figure 7.

The safety needle assembly 101 mounted to the drug delivery device is then arranged on the skin of the patient in a manner that the longitudinal axis A101 extends essentially perpendicularly to the skin of the patient. The base member 104.2 rests on the skin of the patient so that the needle shield 104 may be axially translated from the initial advanced position PI101 in the proximal direction P101 by pushing the drug delivery device towards the skin surface, whereby the compression spring 105 is partially tensioned. The at least one snap 102.3.1 of the longitudinal arm 102.3 protrudes the distal end 102.4 of the needle hub 2. For limiting a needle insertion depth, the needle hub 102 is preferably provided with a radial flange (not shown) that prevents the needle shield 104 from moving in the proximal direction P

The needle shield 104 is now arranged in the retracted position PR101 as illustrated in figure 8. The needle 103 protrudes from a distal end of the safety needle assembly 101 by a length that allows for a disposal of the drug in an appropriate depth. The compression spring 105 is compressed and energized so that the needle shield 104 automatically advances towards the advanced position PA101 illustrated in figure 9 when the drug delivery device with the safety needle assembly 101 mounted thereto is removed from the skin surface of the patient. The needle hub 102 together with the flexible strip 107.1 guided in the integrated guiding track 104.4 now translates relative to the needle shield 104 until the radial protrusions 102.2 of the needle hub 102 engages the proximal end of the slots 104.1 of the needle shield 104.

Upon this translation of the needle shield 104 from the retracted position PR101 to the advanced position PA101 in the distal direction D101, the flexible strip 107.1 is drawn in the proximal direction P101 and thus from the unclosed position PU101 in which the needle aperture 104.3 is aligned with the through hole 107.2 to the closed position PC101 in which the needle aperture 104.3 is misaligned with the through hole 107.2. The wings 107.4 of the distal end of the closure element 107 and thus the needle aperture 104.3 is covered and closed. Furthermore, the wings 107.4 at the distal end of the flexible strip 107.1 relax so as to engage in corresponding recesses 104.4.2 of the guiding track 104.4 to irreversibly lock the flexible strip 107.1 with respect to the needle shield 104.

The safety needle assembly 101 is in a needle safe state when the needle shield 104 reaches the advanced position PA101 as shown in figure 9. The needle shield 104 surrounds the needle 103 and the flexible strip 107.1 covers and closes the needle aperture 104.3 so as to reduce the risk of an accidental needle stick injury caused by the used and thus contaminated needle 103. The safety needle assembly 101 may thus also help to reduce transmission of blood-borne diseases, like for example HIV, AIDS, Hepatitis B or Hepatitis C.

The transverse flap 107.1 is irreversibly positioned with respect to the needle shield 104 over the needle aperture 104.3 and the needle hub 102 is irreversibly positioned with respect to the needle shield 104. Thus, a subsequent proximal translation of the needle shield 104 with respect to the needle hub 102 is blocked. The safety needle assembly 101 is thus irreversibly locked in the advanced position PA101 and may be safely detached from the drug delivery device and disposed after use.

Figures 10 to 12 show isometric longitudinal sections of a safety needle assembly 201 according to a third embodiment of the invention before, during and after use. The safety needle assembly 201 comprises a needle hub 202 mounting a needle 203 and a substantially cylindrical needle shield 204 translatably arranged on the needle hub 202.

In figure 10, the safety needle assembly 201 is arranged in an out of the box state as it would be presented to an end-user. The needle shield 204 is positioned in an initial advanced position PI201, wherein a pointed distal tip 203.1 of the needle 203 is surrounded by the needle shield 204.

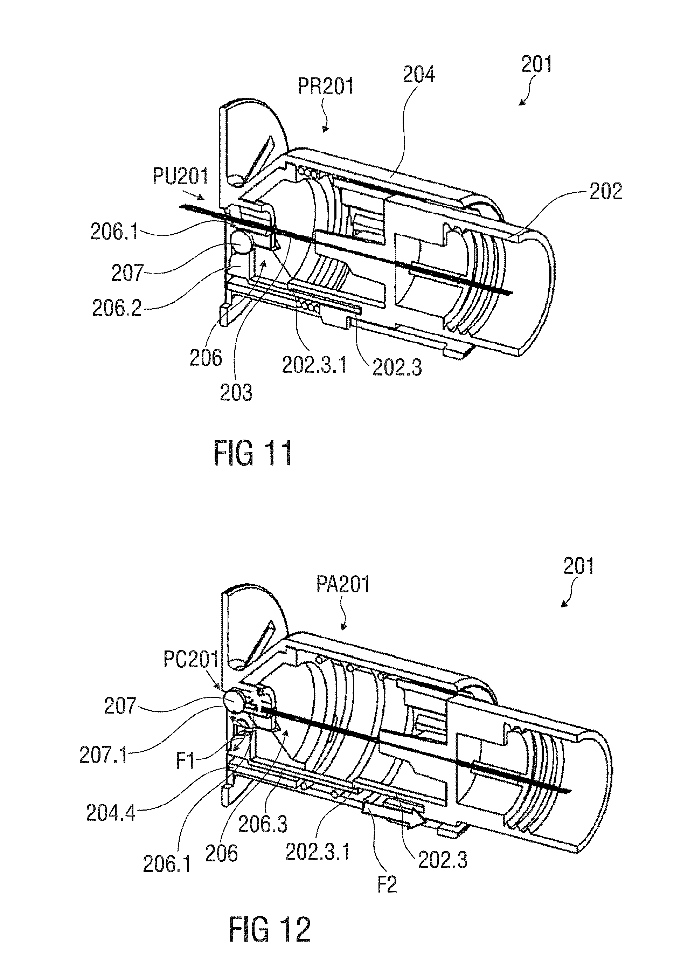

The needle shield 204 is axially translatable relative to the needle hub 202 so as to cover the distal tip 203.1 as in figure 10 or to expose the distal tip 203.1 when a drug is delivered to a patient as in figure 11.

Furthermore, the needle hub 202 is similarly designed as in the previous embodiment shown in figures 1 to 6 or 7 to 9 and also comprises radial protrusions 202.2 arranged around an outer circumference of the needle hub 202 and at equal angular distances to each other. The needle shield 204 comprises corresponding longitudinal slots 204.1 formed into the wall of the needle shield 204. The radial protrusion 202.2 travels along the longitudinal slot 204.1 when the needle shield 204 is translated relative to the needle hub 202 so as to prevent a rotation of the needle shield 204 relative to the needle hub 202.

A spring element 205 arranged as a compression spring biases the needle shield 204 with respect to the needle hub 202 in a distal direction D201.

The needle shield 204 comprises a substantially cylindrical shape which tapers toward the distal direction D201 whereby the distal end of the needle shield 204 has an enlarged base member 204.2. The enlarged base member 204.2 has a needle aperture 204.3 concentrically arranged about the longitudinal axis A201.

Furthermore, the safety needle assembly 201 comprises a further alternative closure element 207. The closure element 207 according to the third embodiment is rotatably arranged with respect to the needle shield 204 between an unclosed position PU201 in which the needle aperture 204.3 is uncovered and open (shown in figures 10 and 11) and a closed position PC201 in which the needle aperture 204.3 is covered and closed by the closure element 207 (shown in figure 12).

The closure element 207 is designed as an essentially spherical ball 207.1 which is guided from the unclosed position PU201 to the closed position PC201. The ball 207.1 is preferably made from a plastic material and is retained by a flip mechanism 206. The flip mechanism 206 is designed as a separate conical sleeve which is non-rotatably arranged in the tapering distal end of the needle shield 204. The flip mechanism 206 comprises an inner bearing surface 206.1 and an inner release flap 206.2. Between them the ball 207.1 is retained before and during drug delivery in the unclosed position PU201 of the needle aperture 204.3 according to figures 10 and 11. Thus the needle aperture 204.3 is open.

Furthermore, the flip mechanism 206 comprises a longitudinal latch arm 206.3 which protrudes the conical sleeve in the proximal direction P201. A proximal end of the latch arm 206.3 comprises a hook adapted to latch to a snap 202.3.1 on a longitudinal arm 202.3 of the needle hub 202 so that the longitudinal arm 202.3 with the snap-in latch arm 206.3 may be drawn in the proximal direction P201 when the needle hub 202 translates relative to the needle shield 204 as indicated by arrow F2 (shown in figure 12) during use of the safety needle assembly 201. Upon this translation the release flap 206.2 on the distal end of the latch arm 206.3 may be released as indicated by arrow F1.

The release flap 206.2 is flexibly arranged on the distal end of the latch arm 206.3. A section between the release flap 206.2 and the latch arm 206.3 is designed as an elastic live hinge made from a resilient plastics material and relatively thin so that a limited pivoting of the release flap 206.2 is allowed when the latch arm 206.3 is axially moved in the proximal direction P202.

Further, the flip mechanism 206 with its conical sleeve, the inner bearing surface 206.1, the inner release flap 206.2 and the latch arm 206.3 are designed as one part, e.g. as an integrally moulded part, especially as an injection-moulded part made from polypropylene.

A drug delivery process involving the safety needle assembly 201 may be carried out as follows: in a first step, the safety needle assembly 201 is attached to a distal end of a housing of a drug delivery device. Initially, the needle shield 204 is retained in the initial advanced position PI201 as illustrated in figure 10.

The safety needle assembly 201 mounted to the drug delivery device is then arranged on the skin of the patient in a manner that the longitudinal axis A201 extends essentially perpendicularly to the skin of the patient. The enlarged base member 204.2 rests on the skin of the patient so that the needle shield 204 may be axially translated from the initial advanced position PI201 in the proximal direction P201 by pushing the drug delivery device towards the skin surface, whereby the compression spring 205 is compressed but the flip mechanism 206 is not activated.

The needle shield 204 is now arranged in the retracted position PR201 as illustrated in figure 11. The needle 203 protrudes from a distal end of the safety needle assembly 201 by a length that allows for a disposal of the drug in an appropriate depth. The compression spring 205 is compressed and energized so that the needle shield 204 automatically advances towards the advanced position PA201 illustrated in figure 12 when the drug delivery device with the safety needle assembly 201 mounted thereto is removed from the skin surface of the patient.

On removal from the skin surface the needle hub 202 translates relative to the needle shield 204 until the radial protrusions 202.2 of the needle hub 202 engage the proximal ends of the slots 204.1 of the needle shield 204. Thus the needle hub 202 is retracted with respect to the needle shield 204. As the needle hub 202 translates the latch arm 206.3 of the flip mechanism 206 lock in the corresponding snap 202.3.1 of the longitudinal arm 202.3 of the needle hub 202 so that the latch arm 206.3 is axially moved in the proximal direction P201 and the flip mechanism 206 is released. Hence the release flap 206.2 is pivoted and releases the ball 207.1. The force from the release flap 206.2 on the ball207.1 causes a deformation of the retention bearing surface 206.1 allowing the ball to be guided past the bearing surface 206.1 and to roll into the closed position PC201 in which the ball 207.1 blocks the needle aperture 204.3 and makes the safety needle assembly 201 safe. The ball 207.1 cannot come out of this closed position PC201 as it is retained between the needle 203, the needle shield 204 and the bearing surface 206.1 (shown in figure 12).

The needle shield 204 surrounds the needle 203 and the ball 207.1 irreversibly covers and closes the needle aperture 204.3 so as to reduce the risk of an accidental needle stick injury caused by the used and thus contaminated needle 203. The safety needle assembly 201 may be safely detached from the drug delivery device and disposed after use.

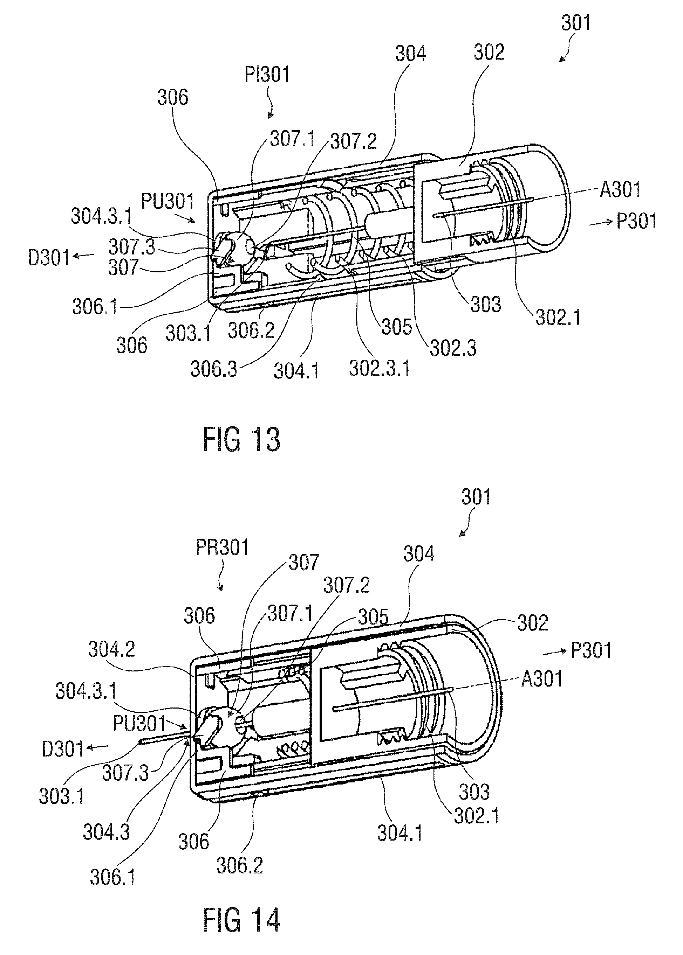

Figures 13 to 15 show sectional and isometric views of a safety needle assembly 301 according to a fourth embodiment of the invention before, during and after use. The safety needle assembly 301 comprises a needle hub 302 mounting a needle 303 and a substantially cylindrical needle shield 304 translatably arranged on the needle hub 302.

In figure 13, the safety needle assembly 301 is arranged in an out of the box state as it would be presented to an end-user. The needle shield 304 is positioned in an initial advanced position PI301, wherein a pointed distal tip 303.1 of the needle 303 is surrounded by the needle shield 304.

The needle shield 304 is axially translatable relative to the needle hub 302 so as to cover the distal tip 303.1 as in figure 13 or to expose the distal tip 303.1 when a drug is delivered to a patient (shown in figure 14).

Furthermore, the needle hub 202 is similarly designed as in the previous embodiment shown in figures 1 to 6 or 7 to 9 or 10 to 12 and also comprises two radial protrusions arranged around an outer circumference of the needle hub 302 and at equal angular distances to each other. These two radial protrusions are not shown in figures 13 to 15. The needle shield 304 comprises corresponding longitudinal slots 304.1 formed in the wall of the needle shield 304. The radial protrusion travels along the longitudinal slot 304.1 when the needle shield 304 is translated relative to the needle hub 302 so as to prevent rotation of the needle shield 304 relative to the needle hub 302.

A spring element 305 arranged as a compression spring biases the needle shield 304 with respect to the needle hub 302 in a distal direction D301.

The needle shield 304 comprises a substantially cylindrical shape and has a base member 304.2. The base member 304.2 has a needle aperture 304.3 concentrically arranged about the longitudinal axis A301.

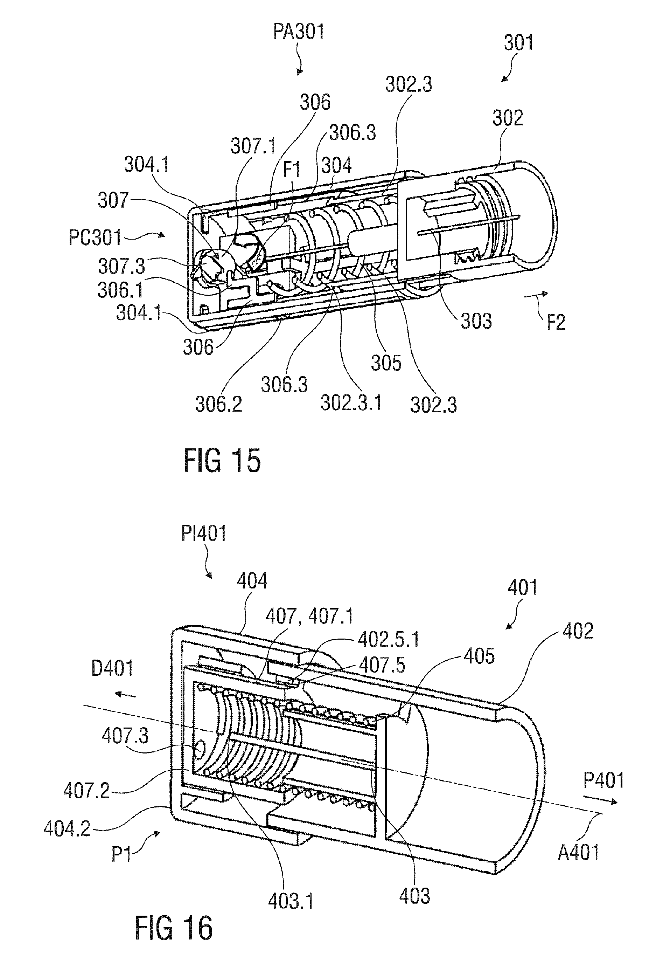

The safety needle assembly 301 comprises a further alternative closure element 307. The closure element 307 according to the fourth embodiment is rotatably arranged with respect to the needle shield 304 between an unclosed position PU301 in which the needle aperture 304.3 is uncovered and open (shown in figures 13 and 14) and a closed position PC301 in which the needle aperture 304.3 is covered and closed by the closure element 307 (shown in figure 15).

The closure element 307 is designed as an essentially spherical ball 307.1 which is rotatably retained by an actuator sleeve 306 above the needle aperture 304.3. The actuator sleeve 306 is designed as a separate sleeve which is non-rotatably arranged in the needle shield 304 by a guide pin slot arrangement. The actuator sleeve 306 comprises at least one actuator finger 306.1 protruding inwards, at least one radial protrusion 306.2 like a pin protruding outwards and at least one latch arm 306.3 protruding in a proximal direction P301.

In the initial state before use of the safety needle assembly 301 the spring 305 is unstressed. The needle aperture 304.3 comprises a circumferential edge 304.3.1 which is adapted to movably hold the ball 307.1, whereby the ball 307.1 is retained in the circumferential edge 304.3.1 slightly stressed by the actuator finger 306.1 to prevent rattling. Furthermore, the ball 307.1 comprises a through hole 307.2 which is aligned with the needle aperture 304.3 in the initial advanced position PI301 (shown in figure 13) and in the retracted position PR301 (shown in figure 14). Thus, the ball 307.1 is retained before and during drug delivery in an unclosed position PU301 of the needle aperture 304.3. The needle aperture 304.3 is opened.

The needle shield 304 comprises the longitudinal slot 304.1 in which the radial protrusion 306.2 is guided when the actuator sleeve 306 is axially translated with respect to the needle shield 304 in the proximal direction A301.

A proximal end of the latch arm 306.3 comprises a hook adapted to latch to a snap 302.3.1 on a longitudinal arm 302.3 of the needle hub 302 so that the longitudinal arm 302.3 with the snap-in latch arm 306.3 may be drawn in the proximal direction P301 when the needle hub 302 translates relative to the needle shield 304 as indicated by arrow F2 (shown in figure 15). Upon this translation the actuator finger 306.1 on the actuator sleeve 306 pivots release arms 307.3 fixed on the ball 307.1 so that the ball 307.1 may be rotated according to arrow F1 and the through hole 307.2 is misaligned with respect to the needle aperture 304.3.

Further, the actuator sleeve 306 with the inwardly directed actuator fingers 306.1, the outwardly directed radial protrusions 306.2 and the latch arm 306.3 are designed as one part, e.g. as an integrally moulded part, especially as an injection-moulded part made from polypropylene.

A drug delivery process involving the safety needle assembly 301 may be carried out as follows: in a first step, the safety needle assembly 301 is attached to a distal end of a housing of a drug delivery device. Initially, the needle shield 304 is retained in the initial advanced position PI301 as illustrated in figure 13.

The safety needle assembly 301 mounted to the drug delivery device is then arranged on the skin of the patient in a manner that the longitudinal axis A301 extends essentially perpendicularly to the skin of the patient. The base member 304.2 rests on the skin of the patient so that the needle shield 304 may be axially translated from the initial advanced position PI301 in the proximal direction P301 by pushing the drug delivery device towards the skin surface, whereby the compression spring 305 is compressed but the actuator sleeve 306 is not activated.

The needle shield 304 is now arranged in the retracted position PR301 as illustrated in figure 14. The needle 303 protrudes from a distal end of the safety needle assembly 301 by a length that allows for a disposal of the drug in an appropriate depth. The compression spring 305 is compressed and energized so that the needle shield 304 automatically advances towards the advanced position PA301 illustrated in figure 15 when the drug delivery device with the safety needle assembly 301 mounted thereto is removed from the skin surface of the patient.

On removal from the skin surface the needle hub 302 translates relative to the needle shield 304 until the radial protrusions of the needle hub 302 engage the proximal ends of the slots 304.1 of the needle shield 304. Thus the needle hub 302 retracts. As the needle hub 302 translates the latch arms 306.3 of the actuator sleeve 306 lock in the corresponding snaps 302.3.1 of the longitudinal arms 302.3 of the needle hub 302 so that the latch arm 306.3 is axially moved in the proximal direction P301 and the actuator sleeve 306 is released, whereby the actuator finger 306.1 pivots the release arms 307.3 fixed on the ball 307.1 and rotates the ball 307.1 into the closed position PC201 such that the through hole 307.2 of the ball 307.1 is misaligned with respect to the needle aperture 304.3. Thus the ball 307.1 blocks the needle aperture 304.3 and makes the safety needle assembly 301 safe. The ball 307.1 cannot come out of this closed position PC301 as a snap feature prevents reopening (not shown).

The needle shield 304 surrounds the needle 303 and the ball 307.1 irreversibly covers and closes the needle aperture 304.3 so as to reduce the risk of an accidental needle stick injury caused by the used and thus contaminated needle 303. The safety needle assembly 301 may be safely detached from the drug delivery device and disposed after use.

Figures 16 to 18 show sectional and isometric views of a safety needle assembly 401 according to a fifth embodiment of the invention before, during and after use. The safety needle assembly 401 comprises a needle hub 402 mounting a needle 403 and a substantially cylindrical needle shield 404 translatably arranged on the needle hub 402.

In figure 16, the safety needle assembly 401 is arranged in a state as it would be presented to an end-user. The needle shield 404 is positioned in an initial advanced position PI401, wherein a pointed distal tip 403.1 of the needle 403 is surrounded by the needle shield 404.

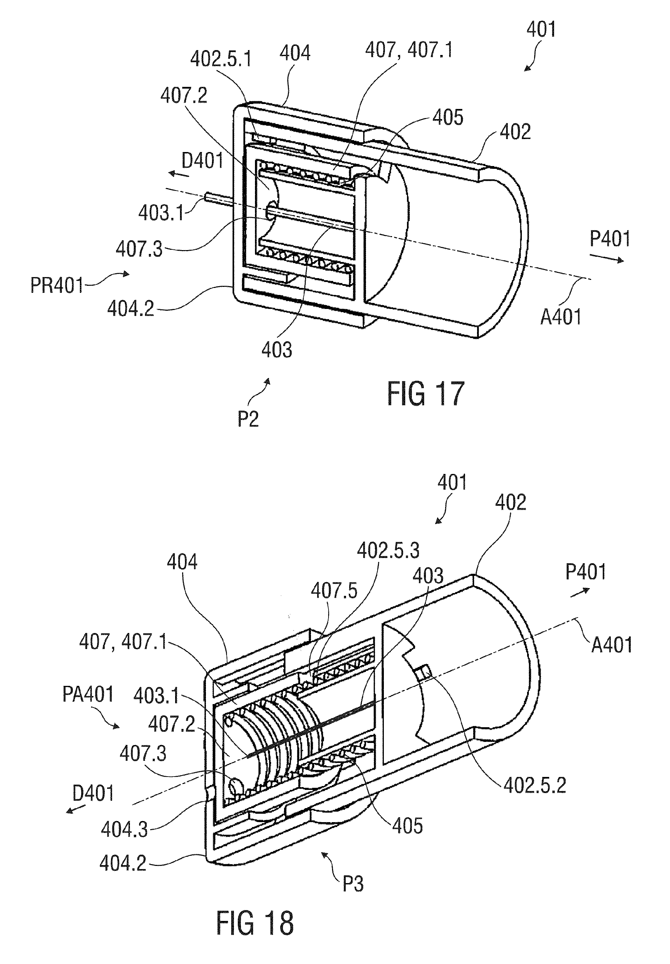

The needle shield 404 is axially translatable relative to the needle hub 402 so as to cover and expose the distal tip 403.1 of the needle 403 when a drug is delivered to a patient (shown in figure 17).

To prevent a rotation of the needle shield 404 relative to the needle hub 402 the needle hub 402 comprises two opposite radial protrusions (not shown) arranged around an outer circumference of the needle hub 402. The needle shield 404 comprises corresponding longitudinal slots (not shown) formed into the wall of the needle shield 404. The radial protrusion travels respectively along one longitudinal slot when the needle shield 404 is translated relative to the needle hub 402.

Further, the needle hub 402 comprises three locking elements 402.5 that are arranged radially on an inner surface of the needle hub 402 and project inwards. In particular, a first locking element 402.5.1 and a second locking element 402.5.2 are designed respectively as a rib, whereby the first locking element 402.5.1 is arranged distally and comprises smaller dimensions than that of the second locking element 402.5.2 which is designed as a longitudinal rib extending parallel to the axis A401 near a distal end towards a proximal end of the needle hub 402. A third locking element 402.5.3 is designed as a longitudinal recess extending parallel to the axis A401.

The needle shield 404 has a first base member 404.2 substantially covering the open distal end of the needle shield 404. The first base member 404.2 has a first needle aperture 404.3 eccentrically arranged with respect to the axis A401.

The term "eccentrically arranged with respect to the axis A401" used herein means that an element is spaced away from the axis A401 in a radial direction that extends perpendicularly to the axis A 401.

The first needle aperture 404.3 has a size corresponding to a width of the needle 403 so that the needle 403 may pass through the first aperture 404.3 and project from the needle shield 404 in a distal direction D.

Furthermore, the safety needle assembly 401 comprises an alternative closure element 407. The closure element 407 according to the fifth embodiment is designed as an inner sleeve 407.1 which is arranged between the needle shield 404 and the needle hub 402 and which is rotatable about the longitudinal axis A401 with respect to the needle shield 404.

The inner sleeve 407.1 has a second base member 407.2 with a second needle aperture 407.3 corresponding to the first needle aperture 404.3 of the needle shield 404. The first and second base members 404.2, 407.2 are arranged parallel to each other. The second needle aperture 407.3 of the inner sleeve 407.1 is eccentrically arranged with respect to the axis A401 so that the needle 403 may protrude through the first and second apertures 404.3, 407.3 when properly aligned.

Thereby, the inner sleeve 407.1 is rotatable with respect to the needle shield 404 from a first angular position P1 in which the first and second needle apertures 404.3, 407.3 are out of alignment (shown in figure 16) to a second angular position P2 in which the first and second needle apertures 404.3, 407.3 are properly aligned (shown in figure 17) further to a third angular position P3 in which the first and second needle apertures 404.3, 407.3 are out of alignment again (shown in figure 18).

The inner sleeve 407.1 further comprises a locking shoulder 407.5 which corresponds to an arrangement of the locking elements 402.5 of the needle hub 402. The locking shoulder 407.5 is arranged proximally and protruding radially outwards. Especially the locking shoulder 407.5 is made from thermoplastic polymer, e.g. polypropylene.

The locking shoulder 407.5 abuts against the first locking element 402.5.1 when the inner sleeve 407.1 is in the first angular position P1, thus preventing a rotation of the inner sleeve 407.1 before use in such a manner that a needle safe state is realized. When the inner sleeve 407.1 is retained in the second angular position P2 the locking shoulder 407.5 abuts against the second locking element 402.5.2 to allow the needle 403 to pass through the first and second apertures 404.3, 407.3 for a drug delivery process. When the inner sleeve 407.1 is retained in the third angular position P3 the locking shoulder 407.5 snaps into the third locking element 402.5.3 irreversibly locking the inner sleeve 407.1 against rotational movement with respect to the needle shield 404 to prevent a re-exposure of the needle 403 and a use of the safety needle assembly 401 a second time. Hence the snap-fit connection of the needle hub 402 and the inner sleeve 407.1 introduces a no-return feature so that the safety needle assembly 101 may only be used once and may be safely detached from a drug delivery device and disposed after use.

Moreover, the safety needle assembly 401 comprises a spring element 405 arranged as a compression spring biasing the needle hub 402 with respect to the needle shield 404, whereby a distal end of the spring element 405 abuts against an inner surface of the base member 407.2. The spring element 405 is arranged as a compression spring. Initially, the spring element 405 is arranged in an unstressed state so that material fatigue of adjacent components of the safety needle assembly 401, in particular the inner sleeve 407.1 and the needle hub 402 is reduced. The spring element 405 is compressed and energized during use of the safety needle assembly 401 by translating the needle shield 404 with respect to the needle hub 402 in the proximal direction P.

Before use, the needle shield 401 is releasably retained in the initial advanced position PI1, wherein the pointed tip 403.1 of the needle 403 is surrounded and shielded by needle shield 404. The inner sleeve 407.1 is in the first angular position P1 as illustrated in figure 16, wherein the first aperture 404.3 and the second aperture 407.3 are out of alignment so that the needle 403 cannot pass there through.

The releasable retention of the needle shield 404 in the initial advanced position PI401 is achieved by snap type coupling of the radial protrusion of the needle hub 402 disposed in the longitudinal slots of the needle shield 404 (not shown).

A drug delivery process involving the safety needle assembly 401 may be carried out as follows: in a first step, the safety needle assembly 401 is attached to a distal end of a housing of a drug delivery device.

The safety needle assembly 401 mounted to the drug delivery device is then arranged on the skin of the patient in a manner that the longitudinal axis A401 extends essentially perpendicularly to the skin of the patient. The base member 404.2 rests on the skin of the patient so that the needle shield 404 may be axially translated from the initial advanced position PI401 in the proximal direction P401 by pushing the drug delivery device towards the skin surface, whereby the spring element 405 is partially tensioned.

The proximal movement of the needle shield 404 allows the locking shoulder 407.5 of the inner sleeve 407.1 to pass the first locking element 402.5.1 proximally, thus releasing the locking arrangement of the inner sleeve 407.1 and the needle hub 402. The spring element 405 now urges the inner sleeve 407.1 to rotate about the axis A until it abuts against the second locking element 402.5.2, hence reaching the second angular position P2 in which the first and second needle apertures 404.3, 407.3 are properly aligned.

The needle shield 404 is now arranged in the retracted position PR401 as illustrated in figure 17. The needle 403 protrudes from a distal end of the safety needle assembly 401 by a length that allows for a disposal of the drug in an appropriate depth. The spring element 405 is compressed and energized so that the needle shield 404 automatically advances towards the advanced position PA401 illustrated in figure 18 when the drug delivery device with the safety needle assembly 401 mounted thereto is removed from the skin surface of the patient.

Upon this translation of the needle shield 404 from the retracted position PR401 to the advanced position PA401 in the distal direction D401, the biasing force provided by the relaxing spring element 405 is now partially transformed into a torque causing the inner sleeve 407.1 to rotate within the needle shield 404.

Because the material of the locking shoulder 407.5 is made from a resilient plastics material allowing a limited elastic deformations, the locking shoulder 407.5 is able to snap into the third locking element 402.5.3 and thus inner sleeve 407.1 is retained in the third angular position P3 in which the needle apertures 404.3, 407.3 are out of alignment and thus the first needle aperture 404.3 is covered and closed.

The safety needle assembly 401 is in a needle safe state when the needle shield 404 reaches the advanced position PA401 as shown in figure 18. The needle shield 404 surrounds the needle 403 and the second base member 407.2 of the inner sleeve 407.1 covers and closes the needle aperture 404.3 so as to reduce the risk of an accidental needle stick injury caused by the used and thus contaminated needle 403. The safety needle assembly 401 may thus also help to reduce transmission of blood-borne diseases, like for example HIV, AIDS, Hepatitis B or Hepatitis C.

The locking shoulder 407.5 is irreversibly positioned in the longitudinal recess of the third locking element 402.5.3. Thereby, the needle shield 404 is locked against a proximal movement by the pointed distal tip 403.1 of the needle 403 hitting the inner surface of the base member 407.2 of the inner sleeve 407.1.

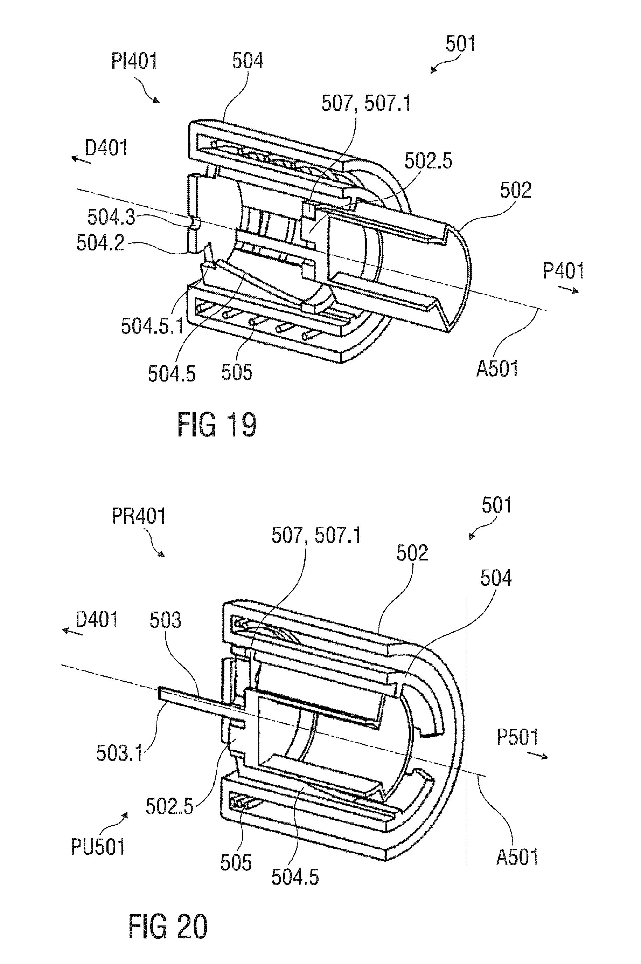

Figures 19 to 21 show sectional and isometric views of a safety needle assembly 501 according to a sixth embodiment of the invention before, during and after use. The safety needle assembly 501 comprises a needle hub 502 mounting a needle 503 and a substantially cylindrical needle shield 504 translatably arranged on the needle hub 502.

In figure 19, the safety needle assembly 501 is arranged in a state as it would be presented to an end-user. The needle shield 504 is positioned in an initial advanced position PI501, wherein a pointed distal tip 503.1 of the needle 503 is surrounded by the needle shield 504.

The needle shield 504 is axially translatable relative to the needle hub 502 so as to cover and expose the distal tip 503.1 of the needle 503 when a drug is delivered to a patient (shown in figure 20).

To prevent a rotation of the needle shield 504 relative to the needle hub 502 the needle hub 502 comprises two opposite radial protrusions (not shown) arranged around an outer circumference of the needle hub 502. The needle shield 504 comprises corresponding longitudinal slots (not shown) formed into the wall of the needle shield 504. The radial protrusion travels respectively along one longitudinal slot when the needle shield 504 is translated relative to the needle hub 502.

The needle hub 502 further comprises a distal protrusion 502.5 projecting inwards from a distal end of the needle hub 502 in the distal direction D501.

The needle shield 404 has a base member 504.2 substantially covering the open distal end of the needle shield 504. The base member 504.2 has a first needle aperture 504.3 concentrically arranged with respect to the axis A501.

The first needle aperture 504.3 has a size corresponding to a width of the needle 503 so that the needle 503 may pass through the first aperture 504.3 and project from the needle shield 504 in a distal direction D501.

The needle shield 504 further comprises an inclined section 504.5 formed on an inner surface of the needle shield 504, whereby the inclined section 504.5 is formed as a ramp extending from a proximal end to a distal end of the needle shield 504. The inclined section 504.5 includes a clearance 504.5.1 arranged distally and forming a latching element.

Furthermore, the safety needle assembly 501 comprises an alternative closure element 507. The closure element 507 according to the sixth embodiment is designed as a spring cover 507.1 which is arranged between the needle shield 504 and the needle hub 502 and which is axially movable with respect to the needle shield 504 or the needle hub 502.

Especially, the spring cover 507.1 comprises a slot 507.2 with a first end and a second end (not shown in detail), whereby the first end is centrically arranged within the spring cover 507.1. The slot 507.2 extends radially outwards. Thereby, an ambient area of the second end of the slot 507.2 is designed as a first substantially part-circle section. An ambient area of the first end of the slot 507.2 is designed as a second substantially part-circle section. The slot 507.2 has a width corresponding to a width of the needle 503 so that the needle 503 may pass through the slot 507.2.

Preferably, the spring cover 507.1 is made from a plastic material of suitable resiliency, especially from thermoplastic polymer, e.g. polypropylene. Although, the thickness of the material has to be great enough that the pointed distal tip 503.1 of the needle 503 is unable to penetrate through an inner surface of the spring cover 507.1.

Thereby, the spring cover 507.1 is axially movable with respect to the needle shield 504 from an unclosed position PU to a closed position PC in which a clearance 504.5.1 in the needle shield 404 allows the spring cover 507.1 to relax and close the first needle aperture504.3.

Moreover, the safety needle assembly 501 comprises a spring element 505 arranged as a compression spring biasing the needle hub 502 with respect to the needle shield 504. The spring element 505 is arranged as a compression spring.

Before use, the needle shield 504 is releasably retained in the initial advanced position PI501, wherein the pointed tip 503.1 of the needle 503 is surrounded and shielded by the needle shield 504 as illustrated in figure 19.

The spring cover 507.1 is releasably engaged with the needle hub 502 on a distal end thereof by a snap-fit connection of the distal protrusion 502.5 of the needle hub 502 and a corresponding design of a outer circumference of the spring cover 507.1, in particular a detend formed between the first part-circle and the second part-circle. The needle 503 protrudes through the slot 507.2 of the spring cover 507.1. Thus, the spring cover 507.1 is in the unclosed position PU.

The releasable retention of the needle shield 504 in the initial advanced position PI501 is achieved by snap type coupling of the radial protrusion of the needle hub 502 disposed in the longitudinal slots of the needle shield 504 (not shown).

The spring element 505 is arranged in an unstressed state so that material fatigue of adjacent components of the safety needle assembly 501, in particular the needle shield 504 and the needle hub 502 is reduced. The spring element 505 is compressed and energized during use of the safety needle assembly 501 by translating the needle shield 504 with respect to the needle hub 502 in the proximal direction P.

A drug delivery process involving the safety needle assembly 501 may be carried out as follows: in a first step, the safety needle assembly 501 is attached to a distal end of a housing of a drug delivery device.

The safety needle assembly 501 mounted to the drug delivery device is then arranged on the skin of the patient in a manner that the longitudinal axis A501 extends essentially perpendicularly to the skin of the patient. The base member 504.2 rests on the skin of the patient so that the needle shield 504 may be axially translated from the initial advanced position PI501 in the proximal direction P501 by pushing the drug delivery device towards the skin surface, whereby the spring element 505 is partially tensioned.

Upon translation of the needle shield 504 relative to the needle hub 502 the spring cover 507.1 is stressed and thus deformed by being guided on the inclined section 504.5 of the needle shield 504 until it passes the clearance 504.5.1, whereby a clearance 504.5.1 in the needle shield 504 allows the spring cover 507.1 to relax partially. The needle 503 protruding through the slot 507.2 when the needle shield 504 is in the retracted position PR501 prevents the spring cover 507.1 fully relaxing and thus covering the first needle aperture 504.3 during drug delivery. At the same time, the engagement of the needle hub 502 with the spring cover 507.1 is released, whereby the spring cover 507.1 is now engaged with the needle shield 504 and irreversibly locked for axially movement with respect to the needle shield 504 by the clearance 504.5.1 designed as an undercut.

The needle shield 504 is now arranged in a retracted position PR501 as illustrated in figure 20. The needle 503 protrudes from a distal end of the safety needle assembly 501 through the slot 507.2 and the first needle aperture 504.3 by a length that allows for a disposal of the drug in an appropriate depth.

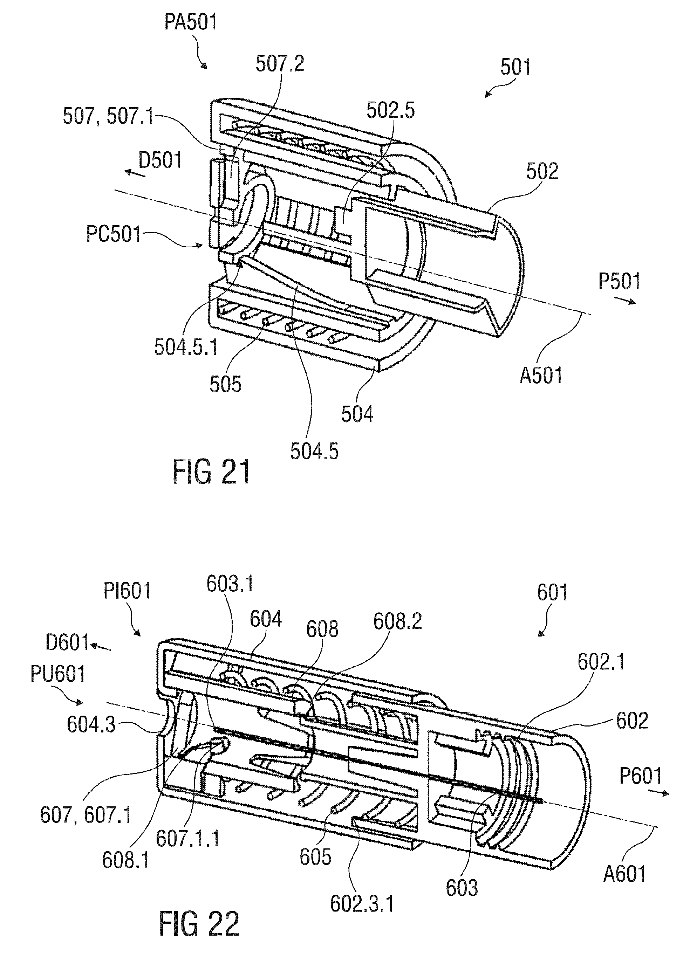

The spring element 505 is compressed and energized so that the needle shield 504 automatically advances towards the advanced position PA501 illustrated in figure 21 when the drug delivery device with the safety needle assembly 501 mounted thereto is removed from the skin surface of the patient.

Upon this translation of the needle shield 504 from the retracted position PR501 to the advanced position PA501 in the distal direction D501, the needle 503 is retracted out from the slot 507.2 allowing the spring cover 507.1 to relax fully and return to its original shape by which the slot 507.2 is out of alignment with the first needle aperture 504.3.

Thereby, the spring cover 507.1 moves from the unclosed position PU to the closed position PC in which the first needle aperture 504.3 is covered and closed.

The safety needle assembly 501 is in a needle safe state when the needle shield 504 reaches the advanced position PA501 as shown in figure 21. The needle shield 504 surrounds the needle 503 and the spring cover 507.1 covers and closes the first needle aperture 504.3 so as to reduce the risk of an accidental needle stick injury caused by the used and thus contaminated needle 503. The safety needle assembly 501 may thus also help to reduce transmission of blood-borne diseases, like for example HIV, AIDS, Hepatitis B or Hepatitis C.

Figures 22 to 24 show sectional and isometric views of a safety needle assembly 601 according to a seventh embodiment of the invention before, during and after use. The safety needle assembly 601 comprises a needle hub 602 mounting a needle 603 and a substantially cylindrical needle shield 604 translatably arranged on the needle hub 602.

In figure 22, the safety needle assembly 601 is arranged in a state as it would be presented to an end-user. The needle shield 604 is positioned in an initial advanced position PI601, wherein a pointed distal tip 603.1 of the needle 603 is surrounded by the needle shield 604.

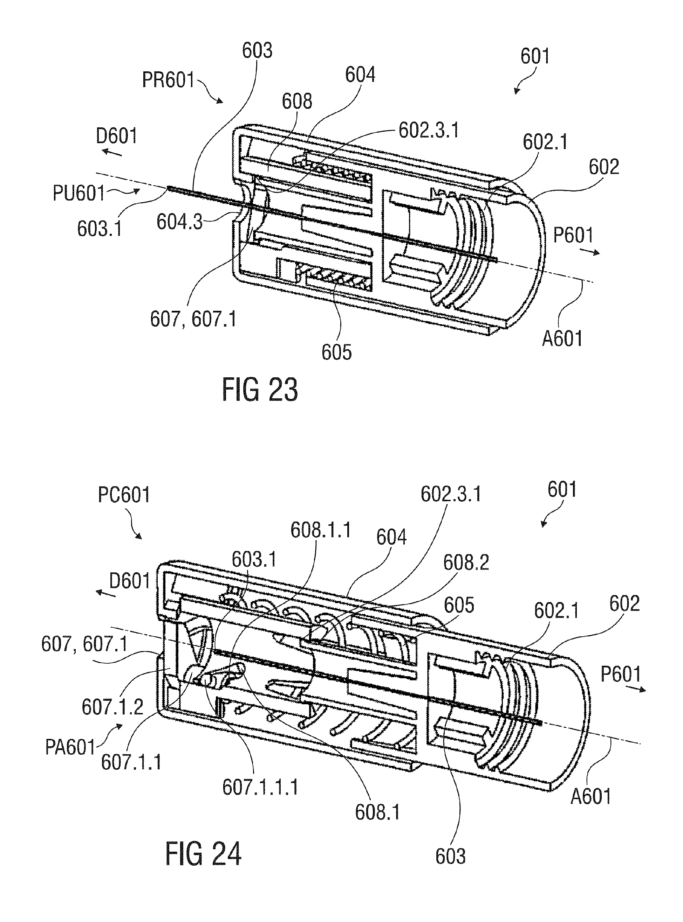

The needle shield 604 is axially translatable relative to the needle hub 602 so as to cover and expose the distal tip 603.1 of the needle 603 when a drug is delivered to a patient (shown in figure 23).

Furthermore, the needle hub 602 is designed as in the first embodiment shown in figures 1 to 6 and also comprises three radial protrusions 602.2 arranged around an outer circumference of the needle hub 602 and at equal distances to each other. The needle shield 604 comprises corresponding longitudinal slots 604.1 formed into the wall of the needle shield 604. The radial protrusion 602.2 travels along the longitudinal slot 604.1 when the needle shield 604 is translated relative to the needle hub 602 so as to prevent a rotation of the needle shield 604 relative to the needle hub 602.

A spring element 605 arranged as a compression spring biases the needle shield 604 with respect to the needle hub 602 in a distal direction D601.

The needle shield 604 has a base member 604.2 substantially covering the open distal end of the needle shield 604. The base member 604.2 has a needle aperture 604.3 concentrically arranged about the longitudinal axis A 601.

Furthermore, the safety needle assembly 601 comprises an alternative closure element 607. The closure element 607 according to the seventh embodiment is designed as a transverse flap 607.1 which is arranged axially affixed within the needle shield 604 and pivotable about a longitudinal axis A601 from an unclosed position PU (shown in figures 22 and 23) to a closed position PC (shown in figure 24), wherein the needle aperture 604.3 is closed. The arrangement of the transverse flap 607.1 and the needle shield 604 may be established by a rotatable plug-in connection.

The transverse flap 607.1 has a substantially L-shaped design with two right angled legs 607.1.1, 607.1.2, whereby a proximal leg 607.1.1 is arranged parallel to the axis A601 and comprises a guide pin 607.1.1.1 designed as a protrusion protruding radially outwards from the proximal leg 607.1.1. The guide pin 607.1.1.1 protrudes into a guiding slot 608.1 formed into a rotation sleeve 608 and designed as an inclined slot. Especially, the guide pin 607.1.1.1 is arranged on a proximal end of the guiding slot 608.1 when the transverse flap 607.1 is in the unclosed position PU and is guided towards a distal end of the guiding slot 608.1 when the transverse flap 607.1 is in the closed position PC.

The locking notch 608.1.1 prevents a re-use of the safety needle assembly 601 after drug delivery. In particular, the locking notch 608.1.1 prevents a further pivoting movement of the guide pin 607.1.1.1 when the transverse flap 607.1 is in the closed position PC. Hence the locking arrangement of the rotation sleeve 608 and the transverse flap 607.1 after drug delivery introduces a no-return feature so that the safety needle assembly 601 may only be used once and may be safely detached from a drug delivery device and disposed after use. Needle stick injuries with contaminated needles 603 may be avoided by the locked transverse flap 607.1.

The rotation sleeve 608 is rotatably arranged between the needle shield 604 and the needle hub 602 and designed as a substantially cylindrical sleeve. Further, the rotation sleeve 608 comprises a snap-fit element 608.2 at a proximal end which corresponds with an annular snap 602.3.1 on a distal end 602.4 of the needle hub 602. The snap-fit element 608.2 of the rotation sleeve 608.1 has a hook-like design and is adapted to latch over the snap 602.3.1 of the needle hub 602 so that the rotation sleeve 608 may be engaged with the needle hub 602 and thus retractable after drug delivery.

Before use, the needle shield 604 is releasably retained in the initial advanced position PI601, wherein the pointed tip 603.1 of the needle 603 is surrounded and shielded by the needle shield 604 as illustrated in figure 22.

The releasable retention of the needle shield 604 in the initial advanced position PI601 is achieved by snap type coupling of the radial protrusion of the needle hub 602 disposed in the longitudinal slots of the needle shield 604 (not shown).

The transverse flap 607.1 is in the unclosed position PU601 so that the needle 603 is allowed to pass through the needle aperture 604.3 when the needle shield 604 is translated from the initial advanced position PI601 to a retracted position PR601.

The rotating sleeve 608 is releasably retained within the needle shield 604, whereby the guide pin 607.1.1.1 is arranged on the proximal end of the guide slot 608.1. Thereby, the rotating sleeve 608 slides over a circular protrusion (not shown) arranged in the needle shield 604. The circular protrusion comprises snap features around its proximal end that prevent the rotating sleeve 608 from being removed.

The spring element 605 is arranged in an unstressed state so that material fatigue of adjacent components of the safety needle assembly 601, in particular the needle shield 604 and the needle hub 602 is reduced. The spring element 605 is compressed and energized during use of the safety needle assembly 601 by translating the needle shield 604 with respect to the needle hub 602 in the proximal direction P.

A drug delivery process involving the safety needle assembly 601 may be carried out as follows: in a first step, the safety needle assembly 601 is attached to a distal end of a housing of a drug delivery device. Initially, the needle shield 604 is retained in the initial advanced position PI601 as illustrated in figure 22.

The safety needle assembly 601 mounted to the drug delivery device is then arranged on the skin of the patient in a manner that the longitudinal axis A601 extends essentially perpendicularly to the skin of the patient. The base member 604.2 rests on the skin of the patient so that the needle shield 604 may be axially translated from the initial advanced position PI601 in the proximal direction P601 by pushing the drug delivery device towards the skin surface, whereby the spring element 605 is partially tensioned.

As the rotating sleeve 608 is made from a resilient plastics material allowing for a limited elastic deformation, the snap-fit element 608.2 is allowed to pass the corresponding snap 602.3.1 when a sufficient axial force is exerted onto the needle shield 604 in the proximal direction P601.

The needle shield 604 is now arranged in the retracted position PR601 as illustrated in figure 23. The needle 603 protrudes from a distal end of the safety needle assembly 601 by a length that allows for a disposal of the drug in an appropriate depth. The compression spring 605 is compressed and energized so that the needle shield 604 automatically advances towards the advanced position PA601 illustrated in figure 24 when the drug delivery device with the safety needle assembly 601 mounted thereto is removed from the skin surface of the patient. The needle hub 602 together with the rotating sleeve 608 now translates relative to the needle shield 604 in the proximal direction P601 until the guide pin 607.1.1.1 reaches the distal end of the guiding slot 608.1 of the retracted rotating sleeve 608.

Upon this translation of the needle shield 604 from the retracted position PR601 to the advanced position PA601 in the distal direction D601, the guide pin 607.1.1.1 is guided in the guiding slot 608.1 causing a rotation of the rotation sleeve 608 and thus a pivoting of the transverse flap 607.1 from the unclosed position PU601 to the closed position PC, wherein the needle aperture 604.3 is closed by the distal leg 607.1.2 of the transverse flap 607.1.

The safety needle assembly 601 is in a needle safe state when the needle shield 604 reaches the advanced position PA601 as shown in figure 24. The needle shield 604 surrounds the needle 603 and the transverse flap 607.1 covers and closes the needle aperture 604.3 so as to reduce the risk of an accidental needle stick injury caused by the used and thus contaminated needle 603. The safety needle assembly 603 may thus also help to reduce transmission of blood-borne diseases, like for example HIV, AIDS, Hepatitis B or Hepatitis C.

The transverse flap 607.1 is irreversibly locked in the guiding slot 608.1 by the locking notch 608.1.1 and the rotation sleeve 608 is fully withdrawn. Thus, a subsequent proximal translation of the needle shield 604 with respect to the needle hub 602 is blocked. The safety needle assembly 604 is thus irreversibly locked in the advanced position PA601 and may be safely detached from the drug delivery device and disposed after use.

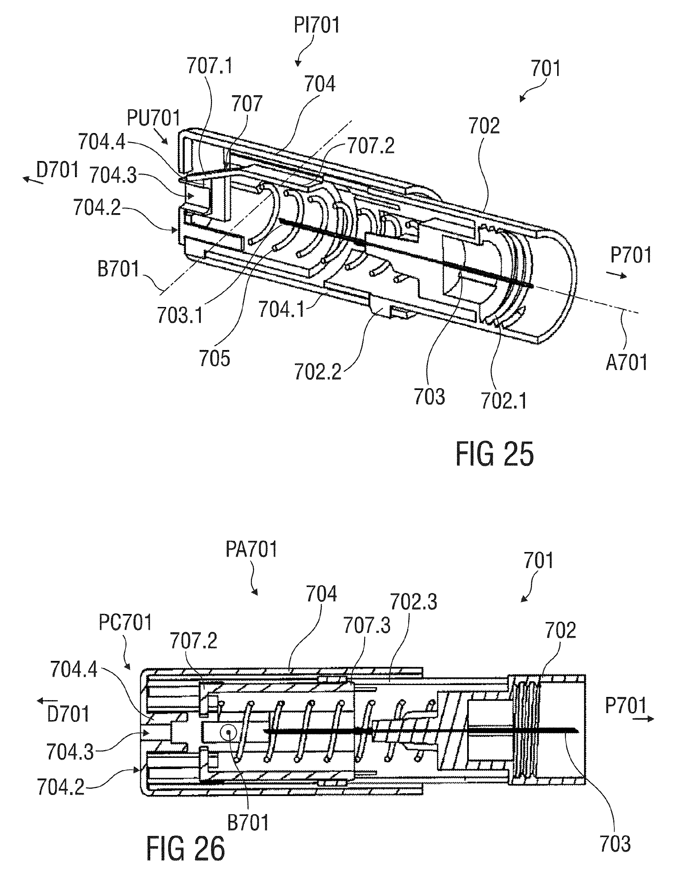

Figures 25 to 28 show sectional and isometric views of a safety needle assembly 701 according to an 8th embodiment of the invention before, during and after use. The safety needle assembly 701 comprises a needle hub 702 mounting a needle 703 and a substantially cylindrical needle shield 704 translatably arranged on the needle hub 702.

In figure 25, the safety needle assembly 701 is arranged in a state as it would be presented to an end-user. The needle shield 704 is positioned in an initial advanced position PI701, wherein a pointed distal tip 703.1 of the needle 703 is surrounded by the needle shield 704 (shown in figure 25).

The needle shield 704 is axially translatable relative to the needle hub 702 so as to cover and expose the distal tip 703.1 of the needle 703 when a drug is delivered to a patient (shown in figure 26).

Furthermore, the needle hub 702 is similarly designed as in the previous embodiment shown in figures 1 to 6, 7 to 9, 10 to 12, etc. and also comprises radial protrusions 702.2 arranged around an outer circumference of the needle hub 702 and at equal distances to each other. The needle shield 704 comprises corresponding longitudinal slots 704.1 formed into the wall of the needle shield 704. The radial protrusion 702.2 travels along the longitudinal slot 704.1 when the needle shield 704 is translated relative to the needle hub 702 so as to prevent a rotation of the needle shield 704 relative to the needle hub 702.

A spring element 705 arranged as a compression spring biases the needle shield 704 with respect to the needle hub 702 in a distal direction D701.

The needle shield 704 has a base member 704.2 substantially covering the open distal end of the needle shield 704. The base member 704.2 has a needle aperture 704.3 concentrically arranged about the longitudinal axis A701.

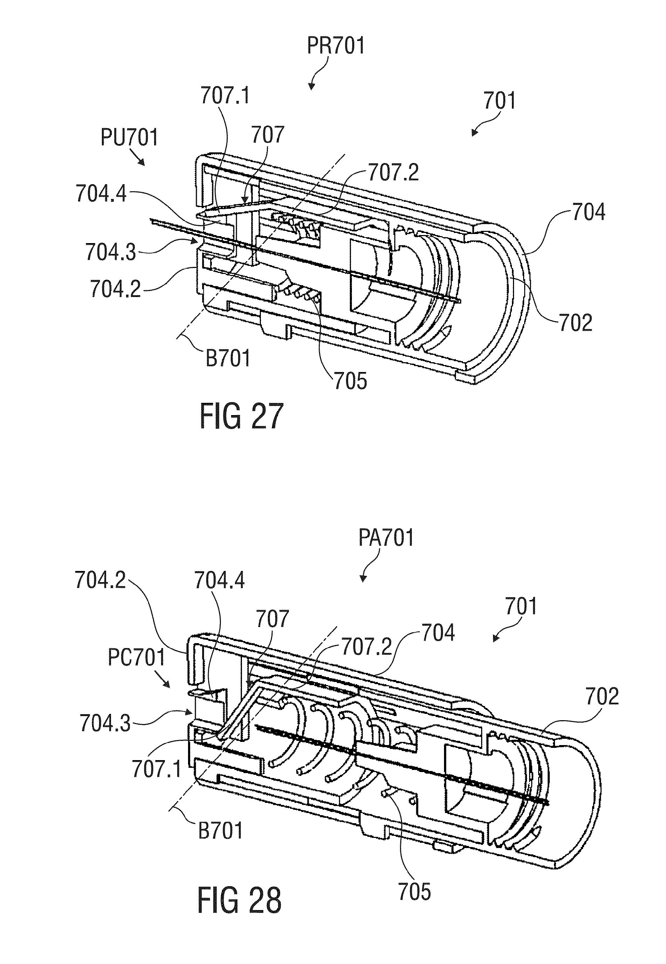

Furthermore, the safety needle assembly 701 comprises an alternative closure element 707. The closure element 707 according to the 8th embodiment is pivoted about a transversal axis B701 with respect to the needle shield 704 from an unclosed position PU701 in which the needle aperture 704.3 is uncovered and opened (shown in figures 25, 27) to a closed position PC701 in which the needle aperture 704.3 is covered and closed by the closure element 707 (shown in figures 26, 28).

The closure element 707 is designed as a flap 707.1. The flap 707.1 is made from a plastic material of suitable resiliency so as to provide a bending or pivoting of a section of the flap 707.1. Especially the flap 707.1 is made from thermoplastic polymer, e.g. polypropylene.

The flap 707.1 is arranged on or part of a substantially cylindrical slider 707.2. In the initial advanced position PI701 of the needle shield 704 a distal end of the flap 707.1 is engaged on an inclined bearing surface 704.4 arranged around the needle aperture 704.3 as a circumferential edge so that the needle aperture 704.3 is opened. The inclined bearing surface 704.4 is designed as a retaining ramp to retain the flap 707.1 in a pre-stressed state in the unclosed position PU701 prior to use.

Figure 26 shows the safety needle assembly 701 turned at 90° opposite the assembly 701 shown in figure 25 and the needle shield 704 in the advanced position PA701 in which the needle aperture 704.3 is covered.

The slider 707.2 comprises at least one snap-fit element 707.3, e.g. two snap-fit elements 707.3, protruding outwards at a proximal end of the slider 707.2. The snap-fit elements 707.3 are adapted to slide in corresponding recesses 702.3 formed into an outer wall of the needle hub 702. The snap-fit element 707.3 of the slider 707.2 has a hook-like design and is adapted to latch into the recess 702.3 of the needle hub 702 so that the slider 707.2 may be drawn in the proximal direction P701 when the needle hub 702 relatively translates to the needle shield 704 during use of the safety needle assembly 701 (shown in figures 26 and 28).

When the needle shield 704 is pressed on to the injection site it is relatively translated with respect to the needle hub 702 to the proximal direction P701 into the retracted position PR701 in which the needle 703 is exposed and the spring 705 is compressed and energized during the proximal movement so that the needle shield 704 is biased in the distal direction D701 (shown in figure 27).

Further, this translation causes that the snap-fit elements 707.3 of the slider 707.2 latch the recesses 702.3 of the needle hub 702. After drug delivery the needle hub 702 relatively translates with respect to the needle shield 704 in the proximal direction P701 by relaxing of spring 705 and pulls the slider 707.2 in the proximal direction P701 so that the flap 707.1 releases, springs and pivots from the bearing surface 704.4 of the needle shield 704 into the closed position PC701 and closes the needle aperture 704.3 (shown in figure 28).

The transverse flap 707.1 is irreversibly positioned with respect to the needle shield 704 over the needle aperture 704.3. The needle 703 is unable to pass through the needle shield 704 and the safety needle assembly 701 can not be used a second time. Any further attempts to expose the needle 703 have the effect of pushing the flap 707.1 which completely closes the needle aperture 704.3 by engaging the outer edge of the bearing surface 704.4. Thus, the safety needle assembly 701 may only be used once and may be safely detached from a drug delivery device and disposed after use. Needle stick injuries with contaminated needles 703 may be avoided by the locking flexible strip 707.1.

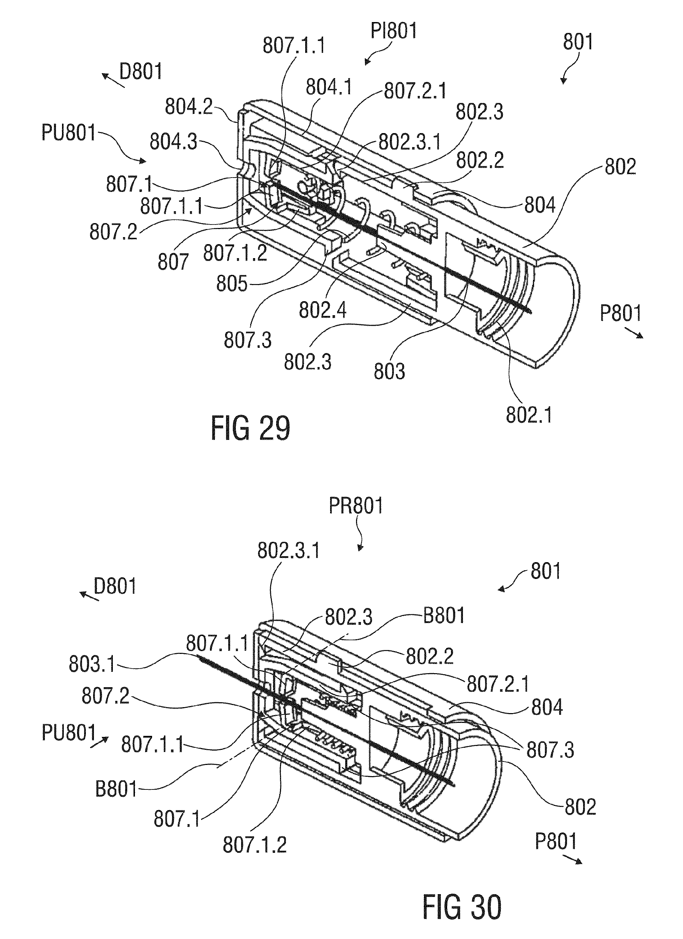

Figures 29 to 31 show sectional and isometric views of a safety needle assembly 801 according to a 9th embodiment of the invention before, during and after use. The safety needle assembly 801 comprises a needle hub 802 mounting a needle 803 and a substantially cylindrical needle shield 804 translatably arranged on the needle hub 802.

In figure 29, the safety needle assembly 801 is arranged in a state as it would be presented to an end-user. The needle shield 804 is positioned in an initial advanced position PI801, wherein a pointed distal tip 803.1 of the needle 803 is surrounded by the needle shield 804.

The needle shield 804 is axially translatable relative to the needle hub 802 so as to cover and expose the distal tip 803.1 of the needle 803 when a drug is delivered to a patient (shown in figure 30).

Furthermore, the needle hub 802 is similarly designed as in the previous embodiment shown in figures 1 to 6, 7 to 9, 10 to 12, etc. and also comprises radial protrusions 802.2 arranged around an outer circumference of the needle hub 802 and at equal distances to each other. The needle shield 804 comprises corresponding longitudinal slots 804.1 formed into the wall of the needle shield 804. The radial protrusion 802.2 travels along the longitudinal slot 804.1 when the needle shield 804 is translated relative to the needle hub 802 so as to prevent a rotation of the needle shield 804 relative to the needle hub 802. The radial protrusions 802.2 are arranged on longitudinal arms 802.3 which protrude from the needle hub 802.

A spring element 805 arranged as a compression spring biases the needle shield 804 with respect to the needle hub 802 in a distal direction D801.

The needle shield 804 has a base member 804.2 substantially covering the open distal end of the needle shield 804. The base member 804.2 has a needle aperture 804.3 concentrically arranged about the longitudinal axis A801.

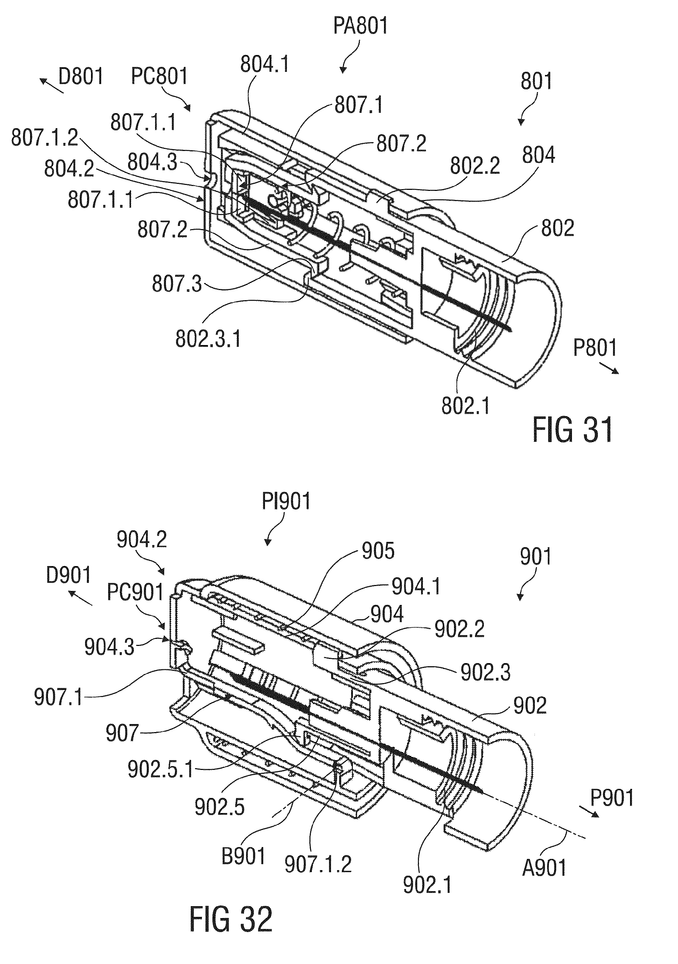

Furthermore, the safety needle assembly 801 comprises an alternative closure element 807. The closure element 807 according to the 9th embodiment is designed as a wing flap 807.1 with oppositely mounted wings 807.1.1 which are pivoted about respective transversal axes B801 with respect to the needle shield 804 from an unclosed position PU801 in which the needle aperture 804.3 is uncovered and opened (shown in figures 29, 30) to a closed position PC801 in which the needle aperture 804.3 is covered and closed by the closed wings 807.1.1 the closure element 807 (shown in figure 31).

The opposite mounted wings 807.1.1 are identically designed. The wing flap 807.1 is arranged on or is part of a locking clamp 807.2. The locking clamp 807.2 has an U-shaped design and comprises a number of locking arms 807.2.1 which corresponds with the number of wings 807.1.1.

Each wing 807.1.1 comprises an integrated spring 807.1.2 which bias the wing 807.1.1 against the locking clamp 807.2, especially against the respective locking arm 807.2.1 to hold the wing flap 807.1 in the unclosed position PU801 before using and thus in an open position so that a passage for the needle 803 is built, as shown in figure 29.

The locking clamp 807.2 comprises at least one snap-fit element 807.3, e.g. two snap-fit elements 807.3, arranging from the proximal end of its locking arms 807.2.1. The snap-fit elements 807.3 are adapted to slide in corresponding snaps 802.3.1 formed at the distal end of longitudinal arms 802.3 protruding from the distal end of the needle hub 802. The snap-fit element 807.3 of the locking clamp 807.2 has a hook-like design and is adapted to latch the snaps 802.3.1 of the needle hub 802 so that the locking clamp 807.2 may be drawn in the proximal direction P801 when the needle hub 802 relatively translates to the needle shield 804 during use of the safety needle assembly 801 (shown in figure 31).

When the needle shield 804 is pressed on to the injection site it is relatively translated with respect to the needle hub 802 to the proximal direction P801 into the retracted position PR801 in which the needle 803 is exposed and the spring 805 is compressed and energized during the proximal movement so that the needle shield 804 is biased in the distal direction D801 (shown in figure 30).

Further, this translation causes that the snap-fit elements 807.3 of the locking clamp 807.2 latch the snaps 802.3.1 of the needle hub 802. After drug delivery the needle hub 802 together with the snapped locking clamp 807.2 relatively translates with respect to the needle shield 804 in the proximal direction P801 by relaxing of spring 805, whereby the wing flap 807.1 especially each wing 807.1.1 is relatively guided in an essentially longitudinal direction with respect to the locking clamp 807.2 on a respective locking arm 807.2.1. Thus the locking clamp 807.2 pulls in the proximal direction P801 so that the wing flaps 807.1 are axially guided on inner surfaces of the locking arms 807.2.1 and on distal inclined sections 807.2.1.1 of the inner surfaces of the locking arms 807.2.1 the opposite wings 807.1.1 are pivoted from unclosed position PU801 (shown in figures 29, 30) to the closed position PC801 so that the ends of the opposite wings 807.1.1 engage each other to close the needle aperture 804.3 (shown in figure 31).

The wings 807.1.1 are held together by the locking arms 807.2.1. This blocks the needle aperture 804.3. The needle 803 is unable to pass through the needle shield 804 and the safety needle assembly 801 can not be used a second time. Any further attempts to expose the needle 803 have the effect of pushing the wings 807.1.1. Thus, the safety needle assembly 801 may only be used once and may be safely detached from a drug delivery device and disposed after use. Needle stick injuries with contaminated needles 803 may be avoided by the locking flexible strip 807.1.

Figures 32 to 34 show sectional and isometric views of a safety needle assembly 901 according to a 10th embodiment of the invention before, during and after use. The safety needle assembly 901 comprises a needle hub 902 mounting a needle 903 and a substantially cylindrical needle shield 904 translatably arranged on the needle hub 902.

In figure 32, the safety needle assembly 901 is arranged in a state as it would be presented to an end-user. The needle shield 904 is positioned in an initial advanced position PI901, wherein a pointed distal tip 903.1 of the needle 903 is surrounded by the needle shield 904.

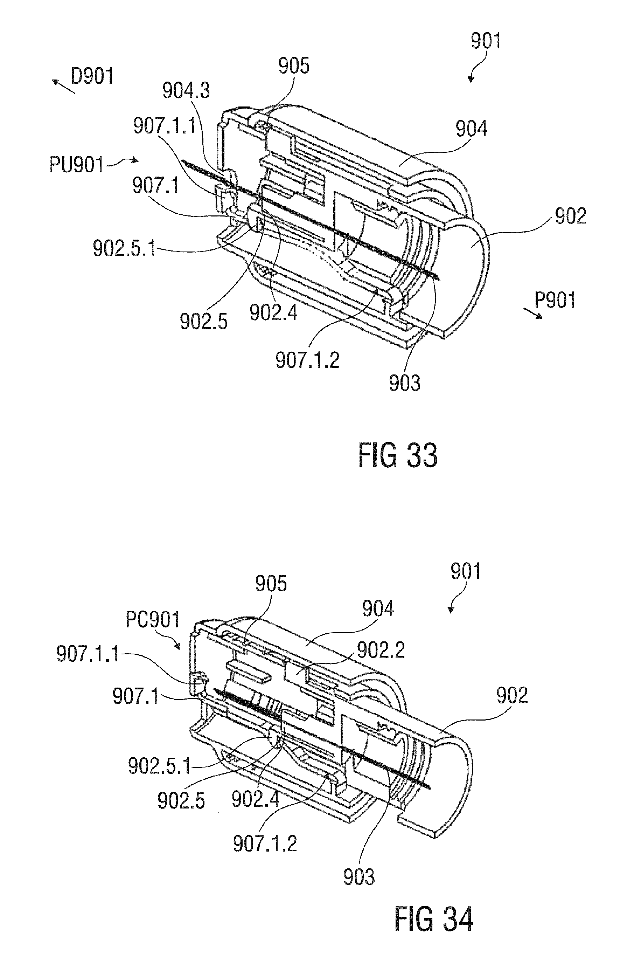

The needle shield 904 is axially translatable relative to the needle hub 902 so as to cover and expose the distal tip 903.1 of the needle 903 when a drug is delivered to a patient (shown in figure 33).

Furthermore, the needle hub 902 is similarly designed as in the previous embodiment shown in figures 1 to 6, 7 to 9, 10 to 12, etc. and also comprises radial protrusions 902.2 arranged around an outer circumference of the needle hub 902 and at equal distances to each other. The needle shield 904 comprises corresponding longitudinal slots 904.1 formed into a wall, e.g. an inner wall, of the needle shield 904. The radial protrusion 902.2 travels along the longitudinal slot 904.1 when the needle shield 904 is translated relative to the needle hub 902 so as to prevent a rotation of the needle shield 904 relative to the needle hub 902. The radial protrusions 902.2 are arranged on longitudinal arms 902.3 which protrude from the needle hub 902.

A spring element 905 arranged as a compression spring biases the needle shield 904 with respect to the needle hub 902 in a distal direction D901.

The needle shield 904 has a base member 904.2 substantially covering the open distal end of the needle shield 904. The base member 904.2 has a needle aperture 904.3 concentrically arranged about the longitudinal axis A901.

Furthermore, the safety needle assembly 901 comprises an alternative closure element 907. The closure element 907 according to the 10th embodiment is designed as at least one blocking arm 907.1 which is pivoted about a respective transversal axis B901 with respect to the needle shield 904 from an initial closed position PC901 in which the needle aperture 904.3 is covered and closed (shown in figure 32) to an unclosed position PU901 in which the needle aperture 904.3 is uncovered and opened (shown in figure 32) back to a closed position PC901 after drug delivery in which the needle aperture 904.3 is closed by a bended distal end 907.1.1 of the blocking arm 907.1 of the closure element 907 (shown in figure 34).

To pivot or flex the blocking arm 907.1 the needle hub 902 comprises an interference arm 902.5 which protrudes over the distal end 902.4 of the needle hub 902 and which comprises at the distal end a radial protrusion 902.5.1.

Prior to drug delivery the needle shield 904 is in its initial advanced position PI901 and covers the needle 903. All flexible parts, e.g. the blocking arm 907.1 and spring 905, are in an unstressed neutral position.

The radial protrusion 902.5.1 of the interference arm 902.5 of the needle hub 902 is adapted to slide on the blocking arm 907.1 when the needle hub 902 is axially translated with respect to the needle shield 904 into the distal direction D901 and to slide in a longitudinal slot 907.1.2 formed into the blocking arm 907.1 when the needle hub 902 is axially translated with respect to the needle shield 904 into the proximal direction P901.

The radial protrusion 902.5.1 has a hook-like design and is adapted to slide on the blocking arm 907.1 so that the interference arm 902.5 may be flexed the blocking arm 907.1 outwards of the needle aperture 904.3 into the uncovered position PU901 when the needle shield 904 relatively translates to the needle hub 902 into the proximal direction P901 during use of the safety needle assembly 901 (shown in figure 33).

Furthermore, the radial protrusion 902.5.1 and the distal end of the slot 907.1.2 of the blocking arm 907.1 are adapted to latch each other so that the blocking arm 907.1 flexes back and thus inwards over the needle aperture 904.3 into the covered position PC901 to irreversibly close and block the needle aperture 904.3. Therefore, the radial protrusion 902.5.1 has a T-shape to ensure that it only latches inside the slot 907.1.2 of the blocking arm 907.1 when reaching the distal end of the blocking arm 907.1.

When the needle shield 904 is pressed on to the injection site the needle shield 904 is relatively translated with respect to the needle hub 902 to the proximal direction P901 into the retracted position PR901 in which the needle 903 is exposed and the spring 905 is compressed and energized during the proximal movement so that the needle shield 904 is biased in the distal direction D901.

Once the needle hub 902 reaches a given injection depth an aperture on the blocking arm 907.1 allows it to pivot back to the closed position PC901. The blocking arm 907.1 rests against the needle 903 until the needle 903 is fully retracted. As soon as the radial protrusions 902.5.1 on the distal end of the interference arm 902.5 passes through the slot 907.1.2 in the blocking arm 907.1 the safety needle assembly 901 is needle-safe. The blocking arm 907.1 is able to flexes back over the needle aperture 904.3 to irreversibly close it whereby the interference arm 902.5 is guided and finally locked in the guiding slot 907.1.2 of the blocking arm 907.1.

The bended distal end 907.1.1 of the blocking arm 907.1 blocks the needle aperture 904.3. The needle 903 is unable to pass through the needle shield 904 and the safety needle assembly 901 can not be used a second time. Thus, the safety needle assembly 901 may be safely detached from a drug delivery device and disposed after use. Needle stick injuries with contaminated needles 903 may be avoided by the blocking arm 907.1.

## Claims

1. Safety needle assembly (1, 101 to 901), comprising
- a needle hub (2, 102 to 902) mounting a needle (3, 103 to 903),
- a needle shield (4, 104 to 904) translatably arranged with respect to the needle hub (2, 102 to 902) and comprising a distal base member (4.2, 104.2 to 904.2) with a needle aperture (4.3, 104.3 to 904.3) through which the needle (3, 103 to 903) may pass,
- a closure element (7, 107 to 907) moveably arranged with respect to the needle shield (4, 104 to 904),
- whereby when the needle shield (4, 104 to 904) is moved from a retracted position (PR, PR101 to PR901) to an advanced position (PA, PA101 to PA901) the closure element (7, 107 to 907) is moved in such a way that it closes the needle aperture (4.3, 104.3 to 904.3).

2. Safety needle assembly (1, 101 to 901) according to claim 1,
wherein the closure element (7, 107 to 907) is arranged inside the safety needle assembly (1, 101 to 901) between at least the needle shield (4, 104 to 904) and the needle hub (2, 102 to 902).

3. Safety needle assembly (1, 201 to 401, 601 to 901) according to claim 1 or 2,
wherein the closure element (7, 207 to 407, 607 to 907) is rotatably arranged with respect to the needle shield (4, 204 to 404, 604 to 904) between an unclosed position (PU, PU201 to PU401, PU601 to PU901) and a closed position (PC, PC201 to PC401, PC601 to PC901).

4. Safety needle assembly (1, 601) according to one of the previous claims,
wherein the closure element (7, 607) is designed as a transverse flap (7.1, 607.1) which is pivoted about a longitudinal axis (A, A601) from the unclosed position (PU, PU601) to the closed position (PC, PC601).

5. Safety needle assembly (1) according to claim 4,
wherein the transverse flap (7.1) comprises an integrated spring arm (7.2) which biases the transverse flap (7.1) relative to a release sleeve (6) and the needle shield (4) in a compressed state so as to releasably retain the transverse flap (7.1) in the unclosed position (PU), whereby when the release sleeve (6) is released upon translation of the needle hub (2) relative to the needle shield (4) the spring arm (7.2) relaxes to pivot the transverse flap (7.1) to the closed position (PC) in which the needle aperture (4.3) is closed.

6. Safety needle assembly (601) according to claim 4,
wherein the transverse flap (607.1) is pivotably mounted on a needle shield (604), whereby upon translation of the needle hub (602) relative to the needle shield (604) a rotation sleeve (608) locks in corresponding snaps (602.3.1) of the needle hub (602) so that the rotation sleeve (608) is axially moved with the needle hub (602) and the flap (607.1) is pivoted by being guided in an inclined slot (608.1) of the rotation sleeve (608) to close the needle aperture (604.3).

7. Safety needle assembly (701) according to one of the previous claims 1 to 3,
wherein the closure element (707) is designed as at least one flap (707.1) which is pivoted about a transversal axis (B701) from the unclosed position (PU701) to the closed position (PC701).

8. Safety needle assembly (701) according to claim 7,
wherein the flap (707.1) is pivotably mounted on a slider (707.2) whereby upon translation of the needle hub (702) relative to the needle shield (704) the slider (707.2) locks in corresponding snaps (702.3.1) of the needle hub (702) so that the slider (707.2) is axially moved with the needle hub (702), wherein the flap (707.1) is pivoted inwards so that the distal end of the flap (707.1) closes the needle aperture (704.3).

9. Safety needle assembly (901) according to one of the previous claims 1 to 3,
wherein the closure element (907) is designed as at least one blocking arm (907.1) which is pivoted about a transversal axis (B901) from the unclosed position (PU901) to the closed position (PC901) and vice versa.

10. Safety needle assembly (901) according to claim 9,
wherein upon translation of the needle hub (902) relative to the needle shield (904) in the distal direction (D901) an interference arm (902.5) arranged on the needle hub (902) flexes the blocking arm (907.1) out of the needle aperture (904.3) to open it and upon translation of the needle hub (902) relative to the needle shield (904) in the proximal direction (P901) the blocking arm (907.1) flexes back over the needle aperture (904.3) to irreversibly close it, whereby the interference arm (902.5) is guided and finally locked in a guiding slot (907.1.2) of the blocking arm (907.1).

11. Safety needle assembly (801) according to one of the previous claims 1 to 3,
wherein the closure element (807) is designed as a wing flap (807.1) with opposite mounted wings (807.1.1) which are pivoted about respective transversal axes (B801) from the unclosed position (PU801) to the closed position (PC801).

12. Safety needle assembly (801) according to claim 11,
wherein each wing (807.1.1) of the wing flap (807.1) is guided in an essentially longitudinal direction on a locking arm (807.2.1) of a U-shaped locking clamp (807.2) whereby upon translation of the needle hub (802) relative to the needle shield (804) proximal ends of the locking arms (807.2.1) lock in corresponding snaps (802.3.1) of the needle hub (802) so that the locking clamp (807.2) is axially moved with the needle hub (802), wherein the wings (807.1.1) are axially guided on an inner surface of the locking arms (807.2.1) and on a distal inclined section of the inner surface of the locking arms (807.2.1) the opposite wings (807.1.1) are pivoted so that the ends of the opposite wings (807.1.1) engage each other to close the needle aperture (804.3).

13. Safety needle assembly (201, 301) according to one of the previous claims 1 to 3,
wherein the closure element (207, 307) is designed as a ball bearing (207.1, 307.1) which is guided from the unclosed position (PU201, 301) to the closed position (PC201, PC301).

14. Safety needle assembly (201) according to claim 13,
wherein the ball bearing (207.1) is retained by a flip mechanism (206) relative to the needle shield (204) in the unclosed position (PU201) whereby when the flip mechanism (206) is released upon translation of the needle hub (202) relative to the needle shield (204) a release flap (206.2) of the flip mechanism (206) is pivoted and releases a ball of the ball bearing (207.1) so that the ball is guided to the closed position (PC201) in which the needle aperture (204.3) is closed.

15. Safety needle assembly (301) according to claim 13,
wherein the ball bearing (307.1) is rotatably retained above the needle aperture (304.3), whereby the ball of the ball bearing (307.1) comprises a through hole (307.2) for the needle (303) and is rotated and guided upon translation of the needle hub (302) relative to the needle shield (304) by actuator fingers (306.1) arranged on an actuator sleeve (306) which is coupled and axially moved with the needle hub (302) to rotate the ball such that the through hole (307.2) is misaligned with respect to the needle aperture (304.3).

16. Safety needle assembly (401) according to one of the previous claim 1 to 3,
wherein the closure element (407) is designed as an inner sleeve (407.1) which is arranged between the needle shield (404) and the needle hub (402) and which is rotatable about a longitudinal axis (A401) with respect to the needle shield (404) from an angular position (P2) in which the needle aperture (404.3) is opened to another angular position (P3) in which the needle aperture (404.3) is closed.

17. Safety needle assembly (401) according to claim 16,
wherein the inner sleeve (407.1) and the needle shield (404) are concentrically arranged about a longitudinal axis (A401) and each of them comprise needle apertures (404.3, 407.3), wherein the needle apertures (404.3, 407.3) and the needle (403) are eccentrically arranged with respect to the longitudinal axis (A401).

18. Safety needle assembly (101) according to claim 1 or 2,
wherein the closure element (107) is drawably arranged with respect to the needle shield (104) between an unclosed position (PU101) and a closed position (PC101).

19. Safety needle assembly (101) according to claim 18,
wherein the closure element (107) is designed as a flexible strip (107.1) which is drawably arranged on an inner surface of the needle shield (104), whereby when the needle hub (102) is relatively moved to the needle shield (102) the flexible strip (107.1) is drawn from the unclosed position (PU101) to the closed position (PC101) in which the needle aperture (104.3) is closed by an essentially transversally guided section of the flexible strip (107.1).

20. Safety needle assembly (101) according to claim 18 or 19,
wherein at least a proximal end of the flexible strip (107.1) and at least a distal end (102.4) of the needle hub (102) comprise corresponding snap-fit elements and the opposite end of the flexible strip (107.1) and a guiding track (104.4) in the needle shield (104) comprise corresponding fixed stop elements to irreversibly lock the flexible strip (107.1) with respect to the needle shield (104) in the closed position (PC101).

21. Safety needle assembly (501) according to claim 1 or 2,
wherein the closure element (507) is designed as a spring cover (507.1) which is arranged between the needle shield (504) and the needle hub (502) and which is axially movable with respect to the needle shield (504) or the needle hub (502) from the unclosed position (PU501) to the closed position (PC501) in which a clearance (504.5.1) in the needle shield (504) allows the spring cover (507.1) to relax and close the needle aperture (504.3).

22. Safety needle assembly (501) according to claim 21,
wherein an inner surface of the needle shield (504) comprises an inclined section (504.5), whereby upon translation of the needle hub (502) relative to the needle shield (504) the spring cover (507.1) is stressed by being guided on the inclined section (504.5) of the needle shield (504).
